# FASCICULE DE BREVET EUROPEEN

(11) **EP 1 200 097 B1**
(45) Date de publication et mention de la délivrance du brevet: **08.01.2014**
(21) Numéro de dépôt: 00925427.7
(22) Date de dépôt: 10.05.2000
(51) Int. Cl.: A61K 31/70, A61K 31/505, A61K 31/55, A61K 31/165, A61K 31/495, A61K 31/66, A61K 31/47

(54) **METHODE POUR CONTROLER LA FIDELITE ET LA PROCESSIVITE DE LA REVERSE TRANSCRIPTASE PAR INCORPORATION ET POLYMERISATION D'ANALOGUES DE NUCLEOTIDES ACCEPTES COMME SUBSTRATS DE LA REACTION DE REVERSE TRANSCRIPTION SANS BLOQUER SON ELONGATION**
METHODE ZUR KONTROLLE DER TREUE UND DER PROZESSIVITÄT DER REVERSTRANSCRIPTASE DURCH INKORPORIERUNG UND POLYMERISIERUNG VON NUCLEOTIDANALOGEN DIE ALS SUBSTRATE DER REVERSTRANSCRIPTIONSREAKTION AKZEPTIERT WERDEN OHNE DIE ELONGIERUNG ZU BLOCKIEREN
METHOD FOR CONTROLLING THE FIDELITY AND THE PROCESSIVITY OF REVERSE TRANSCRIPTASE BY INCORPORATING AND POLYMERISING NUCLEOTIDE ANALOGUES ACCEPTED AS SUBSTRATES OF THE REVERSE TRANSCRIPTION RESPONSE WITHOUT BLOCKING ITS ELONGATION

(30) Priorité: 10.05.1999 FR 9905905
(43) Date de publication de la demande: 02.05.2002
(73) Titulaire: Vigilent Technologies, 38160 Chevrieres (FR)
(72) Inventeur: DERRIEN, Valérie, F-75014 Paris (FR); REISS, Claude, F-91470 Les Molières (FR)
(74) Mandataire: Catherine, Alain
(86) Numéro de dépôt international: PCT/FR2000/001260
(87) Numéro de publication internationale: WO 2000/067698

(56) Documents cités:
- WO-A-96/40166
- WO-A1-96/28162
- WO-A2-94/18979
- WO-A2-97/21452
- MARTINEZ M A ET AL: "REVERSE TRANCRIPTASE AND SUBSTRATE DEPENDENCE OF THE RNA HYPERMUTAGENESIS REACTION" NUCLEIC ACIDS RESEARCH,GB,OXFORD UNIVERSITY PRESS, SURREY, vol. 23, no. 14, 1995, pages 2573-2578, XP000876548 ISSN: 0305-1048
- MCINTOSH, EVAN M. (1) ET AL: "HIV and human endogenous retroviruses: A hypothesis with therapeutic implications." ACTA BIOCHIMICA POLONICA, (1997) VOL. 43, NO. 4, PP. 583-592., XP000878787
- TONG, WENJING ET AL: "Nucleotide-Induced Stable Complex Formation by HIV -1 Reverse Transcriptase" BIOCHEMISTRY (1997), 36(19), 5749-5757, XP002131945
- OSTRANDER, MICHAEL ET AL: "Properties of herpes simplex virus type 1 and type 2 DNA polymerase" BIOCHIM. BIOPHYS. ACTA (1980), 609(2), 232-45 , XP001014122
- LOAKES D ET AL: "ANTIVIRAL ACTIVITY OF BICYCLIC PYRIMIDINE NUCLEOSIDES" ANTIVIRAL CHEMISTRY & CHEMOTHERAPY,GB,BLACKWELL SCIENTIFIC PUBL., LONDON, vol. 6, no. 6, 1995, pages 371-378, XP000671407 ISSN: 0956-3202
- HILL, F. ET AL.: "Comparative mutagenicities of N6-methoxy-2,6-diaminopurine and N6-methoxyaminopurine 2'-deoxyribonucleosides and their 5'-triphosphates" NUCLEIC ACIDS RES., vol. 26, no. 5, - 1998 pages 1144-1149, XP002172901
- KOWALZICK L ET AL: "DIFFERENTIAL INCORPORATION OF THYMIDYLATE ANALOGS INTO DNA BY DNA POLYMERASE ALPHA AND BY DNA POLYMERASES SPECIFIED BY 2 HERPES SIMPLEX VIRUSES." J GEN VIROL, (1982) 62 (PART 1), 29-38. , XP001014120
- DE CLERCQ, ERIK: "Development of resistance of human immunodeficiency virus (HIV) to anti-HIV agents: how to prevent the problem?" INT. J. ANTIMICROB. AGENTS (1997), 9(1), 21-36, XP000878561
- MORRIS-JONES, STEPHEN ET AL: "Antiretroviral therapies in HIV-1 infection" EXPERT OPIN. INVEST. DRUGS (1997), 6(8), 1049-1061, XP002131942
- PATICK, A. K. ET AL: "Activities of the human immunodeficiency virus type 1 (HIV-1) protease inhibitor nelfinavir mesylate in combination with reverse transcriptase and protease inhibitors against acute HIV-1 infection in vitro" ANTIMICROB. AGENTS CHEMOTHER. (1997), 41(10), 2159-2164, XP002131943
- DEBYSER, Z. ET AL: "Antiviral therapy for HIV infection" EOS--RIV. IMMUNOL. IMMUNOFARMACOL. (1996), 16(2), 48-52, XP002131944
- CLERCQ DE E ET AL: "KNOCKING OUT HUMAN IMMUNODEFICIENCY VIRUS THROUGH NON-NUCLEOSIDE REVERSE TRANSCRIPTASE INHIBITORS USED AS SINGLE AGENTS OR IN COMBINATIONS: A PARADIGM FOR THE CURE OF AIDS?" FARMACO,IT,SOCIETA CHIMICA ITALIANA, PAVIA, vol. 50, no. 11, 1 novembre 1995 (1995-11-01), pages 735-747, XP000579600 ISSN: 0014-827X
- GRANIER F. ET AL: "[HIV infections: Contribution of antiretroviral combinations ]. INFECTIONS A VIH: L'APPORT DES COMBINAISONS ANTIRETROVIRALES." PRESSE MEDICALE, (4 APR 1998) 27/13 (622-623)., XP002131947
- BERGMAN A M ET AL: "BIOLOGICAL ACTIVITY OF 1-DEAZAPURINE NUCLEOSIDES: ROLE OF DEOXYCYTIDINE KINASE?", NUCLEOSIDES & NUCLEOTIDES, MARCEL DEKKER, INC, US, vol. 18, no. 4-5, 1 April 1999 (1999-04-01), pages 897-898, XP002997965, ISSN: 0732-8311

## Description

La présente invention concerne notamment le traitement des pathologies induites par les virus rétroïdes notamment HIV 1 en utilisant des analogues nucléotidiques qui module la fidélité et la processivité de la reverse transcriptase de ce virus.

Les virus responsables du syndrome d'immunodéficience acquise (SIDA) ont fait l'objet de nombreux travaux depuis 1981, date à laquelle la maladie a été pour la première fois identifiée. Le SIDA est un problème de santé publique pour beaucoup de pays dans le monde. Ainsi aux USA par exemple, le nombre de cas de SIDA déclarés dépasse les 100 000 et le nombre de personnes infectées a été estimées à plus de un million. La propagation de la maladie est accentuée du fait du nombre de porteurs chroniques du virus responsable du SIDA qui restent asymptomatiques pendant de nombreuses années, sinon leur vie entière, et sont donc des sources de contagion non identifiées. Cette maladie est transmissible par voies sexuelle et sanguine.

Le SIDA est une maladie qui affecte le système immunitaire de l'hôte, favorisant ainsi l'apparition d'infections opportunistes ou de pathologies contre lesquelles un système immunitaire sain aurait protégé l'hôte. Lorsque le SIDA est déclaré, la mort survient généralement deux à trois ans après le diagnostic suite à un effondrement des défenses immunitaires du patient et à de multiples infections opportunistes.

Il est très difficile de classer les virus du SIDA vu l'extrème variabilité génétique et antigénique dont ils font preuve ; on admet classiquement qu'il existe deux types de virus responsables du SIDA humaines : HIV-1 et HIV-2 (Fauci, 1998). Le virus HIV-1 est l'agent responsable du SIDA en Afrique centrale, en Europe, aux USA et dans beaucoup d'autres pays, tandis que HIV-2 est prédominant dans l'ouest de l'Afrique. L'analyse comparative précise de différents génomes viraux a montré que le virus HIV-2 est plus proche des virus simiens du macaque (SIVmac) et du mangabé (SIVsm) qu'il ne l'est du virus humain HIV-1 et de son homologue chez le chimpanzé (SIVcpz). Outre les trois types de virus simiens, il existe des virus félins et chez d'autres mammifères.

Les virus du SIDA font parti du groupe des virus rétroïdes dont la caractéristique commune majeure est d'utiliser au cours du cycle viral et infectif une enzyme très particulière la reverse transcriptase (RT). La caractéristique de cette enzyme est de catalyser la synthèse d'un ADN double brin à partir d'une matrice d'ARN simple brin. Les divers membres du groupe des rétroïdes comportent les rétrovirus (oncogènes et non oncogènes), les hépadnavirus (virus des hépatites) et les caulimovirus (virus des plantes supérieures). Dans le groupe des rétrovirus, on distingue classiquement trois sous-groupes : les oncovirus à ARN, les lentivirus (HIV) et les spumavirus.

Beaucoup d'efforts ont été entrepris pour tenter de comprendre et d'identifier les bases moléculaires de chaque étape du cycle viral de HIV afin de développer des thérapies curatives ou préventives du SIDA. Lé récepteur CD4 est la molécule dont le rôle est prédominant lors de l'infection par le HIV ; ce récepteur est localisé à la surface de plusieurs cellules du sytème immunitaire : les lymphocytes T4 auxiliaires, les monocytes et les macrophages. Cette molécule interagit avec une grande affinité avec des glycoprotéines virales (gp120 pour HIV-1 et gp140 pour HIV-2) enchassées dans l'enveloppe virale externe. Le virus n'entre dans la cellule-cible porteuse du récepteur CD4 qu'en présence d'un cofacteur (par exemple CCR5, CCR3 et CCR2b) qui permet la fusion de l'enveloppe virale avec la membrane cellulaire (Alkhatib et al., 1996 ; Deng et al., 1996, Choe et al., 1996) Lorsque le virus infecte la cellule, son ARN génomique est libéré. La reverse transcriptase (RT) catalyse la synthèse d'ADN proviral dans le cytoplasme dans l'heure qui suit l'infection. Après rétrotranscription, la molécule d'ADN double-brin virale résultante, pénètre dans le noyau de la cellule infectée. L'intégrase P32 clive l'ADN génomique de la cellule hôte permettant ainsi à l'ADN nucléaire ouvert d'acceuillir l'ADN proviral (Kim et al., 1989 ; Sato et al., 1992). Il ne semble pas y avoir à priori de régions d'intégration préférentielles dans les chromosomes des cellules cibles. L'intégration est suivie d'une longue période de latence puis le provirus intégré est exprimé (transcription et traduction de ses gènes). Il est transcrit en ARN qui servira de génome pour de nouveaux virions et également de messager pour la synthèse des protéines virales.

Pour de multiples raisons, le mécanisme de réplication de HIV pose de nombreux problèmes pour l'obtention d'une thérapie efficace. En effet, l'ADN proviral de par son intégration dans le génome cellulaire se comporte comme un élément génétique de l'hôte. D'autre part, le virus HIV est disséminé à travers tout le corps dans les lymphocytes T, les monocytes, les macrophages ainsi que dans le système nerveux central. Enfin, le virus HIV possède une variabilité antigénique extrèmement importante ayant deux causes principales : la faible fidélité de la reverse transcriptase virale (Gojobori et al. (1985) ; Jolly et al. (1986)) qui ne possède pas de mécanisme de correction des erreurs et le taux important de recombinaison du génome viral lié à la nature diploïde de ce même génome (Pathak et al., 1990 a et b ; Hu et Temin, 1990b).

Les diverses thérapies curatives employées actuellement en clinique consistent pour l'essentiel en une attaque frontale du virus soit en bloquant l'activité de la reverse transcriptase, soit en inhibant l'activité d'enzymes virales indispensables pour l'infection ou la réplication (protéases, intégrases). Divers antiviraux traditionnels ont été identifiés par évaluation systématique (criblage) de molécules améliorées par substitutions chimiques successives après démonstration d'un effet antiviral. Plus récemment, la construction à partir de modèles moléculaires cristallographiques d'inhibiteurs viraux en visant une stricte adaptation de leur cible a été explorée. Ces médicaments sont classés selon leur site d'action et leur structure chimique. Les plus utilisés dans les essais cliniques en multithérapie sont des inhibiteurs de la reverse transcriptase nucléotidiques et non nucléotidiques.

Les analogues nucléotidiques développés jusqu'ici interfèrent avec l'infection de HIV et sa réplication de HIV par des incorporations spécifiques inhibitrices de la reverse transcriptase. En effet lorsque la reverse transcriptase incorpore à la chaîne ADN en cours de synthèse des composés chimiques analogues aux nucléotides bloqués en 3', la molécule ainsi néosynthétisé se termine par un site incapable d'accepter l'addition avec un autre nucléotide. L'ADN tronqué a perdu une partie de l'information génétique et presque certainement son infectivité.
Parmi ces agents il faut citer le 3'-azido-3'-déoxythimidine (AZT), le 2',3'-didéoxyinosine (ddI), le 2',3'-didéoxycitidine (ddC), le 2',3'-didéoxy-3'-thiacytidine (3TC), le 2',3'-didéhydro-2',3'-didéoxythymidine (D4T) et le 2'-déoxy-5-fluoro-3'-thiacytidine (FTC). L'utilisation clinique de l'AZT permet de diminuer la fréquence et la gravité des infections opportunistes et de réduire la mortalité chez les patients mais sa toxicité non négligeable en interdit l'utilisation continue. Même si certains analogues s'avèrent moins toxiques, tel que la didéoxyinosine (ddI), tout en étant aussi actifs que l'AZT, l'efficacité de ces agents reste limitée dans la mesure où l'absorption de ces antiviraux entraîne des effets secondaires. De plus, grâce à son fort taux de mutation, le virus HIV devient rapidement résistant à l'AZT et aux autres analogues nucléotidiques durant la thérapie. L'émergence de résistants rend nécessaire l'augmentation des doses thérapeutiques administrées aux patients. Il a ainsi été montré qu'au bout d'un certain temps l'AZT peut inhiber certaines ADN polymérases cellulaires à des concentrations plus faibles que celles inhibant la reverse transcriptase de HIV-1, ce qui explique la toxicité de ces composés nucléotidiques.

Un autre groupe d'inhibiteurs spécifiques de la reverse transcriptase a été découvert plus récemment en utilisant une approche très différente. Alors que la recherche sur les analogues nucléotidiques suit un schéma rationnel basé essentiellement sur les propriétés d'agir comme terminateur de la reverse transcriptase, la découverte des inhibiteurs non nucléotidiques vient de criblages systématiques dans les cellules infectées par le HIV-1, de substances non toxiques pour les cellules non infectées. Le premier inhibiteur découvert fut le TIBO (Tétrahydroimidazo-(4, 5,1-1, jk)(1,4)-benzodiapézine-2(1H)-one) suivi d'autres composés comme le HEPT (1-[(2-hydroxyéthoxy)-méthyl]-6-phénylthiothymine), le TSAO ([2', 5'-bis-O-(tert-butyldiméthylsilyl)-béta-D-ribofuranosyl]-3'-spiro-5"-(4"-amino-1 ", 2"-oxathiole-2", 2"-dioxide) et l'alpha-APA (alpha-anilinophénylacétamide). D'autres inhibiteurs non nucléotidiques comme la névirapine et le BAHP (bis(étheroaryl)-pipérazine) ont été identifiés grâce à leur pouvoir inhibiteur de la reverse transcriptase *in vitro.* Contrairement aux analogues nucléotidiques qui sont également actifs contre d'autres reverse transcriptases, les inhibiteurs non nucléotidiques sont extrêmement spécifiques vis-à-vis de la reverse transcriptase de HIV-1, ils n'affectent pas les polymérases de HIV-2, du virus d'immunodéficience simien SIV, du virus d'immunodéficience du félin FIV et des rétrovirus aviaires et murins.

L'utilisation prolongée des compositions pharmaceutiques actuelles comportant des inhibiteurs nucléotidiques et non nucléotidiques conduit, quel que soit l'inhibiteur utilisé, au développement de rétrovirus variants qui présentent une résistance à ces composés. Ainsi des études cliniques ont montré qu'environ 60 à 75% des patients possèdent, à un stade avancé de la maladie, des variants résistants aux drogues après une année de traitement à l'AZT (Japour *et al,* 1991).

L'échec des thérapies curatives actuelles nécessite donc le développement de nouvelles stratégies thérapeutiques pour lesquelles la reverse transcriptase reste une cible privilégiée pour combattre l'infection rétrovirale. Il est nécessaire de bien comprendre que la faible fidélité de la reverse transcriptase de HIV conduit certes à produire un grand nombre de virions non infectieux, ce qui pour le virus pourrait être considéré comme un handicap (mais néanmoins immunogénique, donc servant de "leurre" pour les lymphocytes circulants), mais que c'est précisément grâce à cette faible fidélité que le virus est capable de produire des souches mutées qui échappent au système immunitaire circulant, ce qui constitue un problème majeur pour mettre au point une stratégie vaccinale ou trouver une thérapie.

En effet, les reverse transcriptases possèdent une fidélité de polymérisation faible due en partie à l'absence de mécanisme de correction d'erreurs (activité proofreading). La fréquence importante d'erreurs introduites par les reverse transcriptases est donc l'une des principales causes des mutations virales. La reverse transcriptase de HIV par exemple fait en moyenne une erreur sur 10000 ce qui a pour conséquence de conférer à la progéniture un taux de mutation élevé et un titre d'infectivité faible dû à la présence d'un grand nombre de mutants non infectieux. Le taux de mutations introduites par la reverse transcriptase de ce virus est de l'ordre de 1000 à 100 000 fois supérieur à celui observé pour les ADN-polymérases cellulaires. Mais si la grande majorité des virions ne sont pas infectieux, ceux qui le sont, sont surtout mutés dans les gènes non vitaux codant pour les protéines immunogènes de l'enveloppe virale, c'est-à-dire les protéines-clé dans une stratégie vaccinale traditionnelle. Ainsi, lors de chaque infection rétrovirale, des millions de variants génétiques peuvent exister en même temps chez un hôte infecté (Domingo et al., 1985) et peuvent ainsi échapper à l'action du système immunitaire en attendant que ce dernier réagisse aux épitopes modifiés. Il s'en suit une spirale mutation d'épitopes viraux/production d'anticorps dirigés contre les épitopes viraux ; le virus, tout en sacrifiant à chaque cycle de reproduction la majeure partie de sa progéniture, produit des variétés virales échappant à la défense immunitaire ambiante car le cycle viral est de moins d'un jour alors que celui de la réponse immunitaire est de plusieurs jours. Cette course-poursuite est d'autant plus à l'avantage du virus que la multiplication des épitopes viraux affaiblit et épuise progressivement l'efficacité de la réponse immunitaire dont les cellules sont la cible du virus.

La présente invention repose sur l'analyse du mode d'action de la reverse transcriptase.

La reverse transcriptase de HIV-1 possède deux sous-unités de 66 et de 51 kDa (Di Marco Veronese et al., 1986). La sous-unité P66 possède les activités polymérasiques et ribonucléasiques. Les données cristallographiques et cinétiques montrent que la reverse transcriptase de HIV-1 présente des degrés de flexibilité au niveau du site catalytique en particulier permettant un jeu assez important dans les positionnements relatifs du déoxyribonucléoside triphosphate (dNTP) en instance d'incorporation, de sa base matrice et de l'extrémité 3'OH du brin naissant. Par ailleurs, la double hélice se présente en conformation A pour les 5 dernières paires de bases formées, en conformation B au delà, avec un coude de 40° entre les deux conformations. Certaines des contraintes de séquences notées ci-dessus donnent lieu à des particularités conformationnelles spontanées permanentes (hélice A pour les suites de plus de 4 à 5 purines, hélice H pour les suites de 3 adénines et plus, coude à la jonction entre les conformations B et A ou B et H). La présence dans les 20 paires de bases couvertes par l'enzyme de l'une ou l'autre de ces contraintes de séquences peut éloigner le dNTP du 3'OH du brin naissant au point d'empêcher l'élongation (perte de processivité), ou encore présenter le dNTP à sa base matrice dans une disposition ne permettant pas l'appariement Watson-Crick complet requis pour la fidélité. Le travail expérimental montre précisément que la majorité des misinsertions sont compatibles avec des appariements Wobble du dNTP avec sa base matrice, qui seraient autorisés par la flexibilité de leur positionnement relatif, déterminé principalement par la conformation de la double hélice engagée dans l'enzyme.

Cette observation permet d'envisager de prendre le contrôle de l'expression et des effets biologiques du fragment d'ADN ou du génome rétrotranscrit par la mise en oeuvre d'analogues de nucléotides bu autres molécules acceptés comme substrats de polymérisation par la reverse transcriptase. La présente invention concerne donc l'utilisation d'analogues nucléotidiques acceptés comme substrat par la reverse transcriptase pour la réalisation d'un médicament destiné au traitement des affections faisant intervenir la reverse transcriptase, caractérisé en ce que lesdits analogues nucléotidiques possèdent un groupement 3'-OH, protégé ou non, situé sur la carbone C3' du 2'-désoxyribose, ledit groupement étant capable d'échanger des liaisons phosphodiester avec la chaîne naissante et le nucléotide suivant, et caractérisé en ce que lesdits analogues nucléotidiques ne terminent pas la réaction de reverse transcription.

Il convient de bien comprendre que les analogues de nucléotides selon l'invention ne sont pas des agents antiviraux immédiats c'est-à-dire stoppant net le cycle viral, mais qu'ils constituent des agents permettant de prendre le contrôle de l'exécution (processivité) et de la précision (fidélité) de la reverse-transcription, rétrovirale en particulier. Les analogues nucléotidiques selon l'invention prennent le contrôle des caractéristiques génétiques du matériel rétro-transcrit.
L'utilisation des analogues nucléotidiques selon l'invention pour la réalisation d'un médicament destiné au traitement des affections faisant intervenir la reverse transcriptase se distingue de celle des analogues nucléotidiques de l'art antérieur par le fait que l'action des analogues nucléotidiques de l'invention est différée et ne provoque pas un blocage et un arrêt immédiat de la synthèse de la chaîne naissante. En effet, les analogues nucléotidiques inhibiteurs de la reverse transcriptase de l'art antérieur utilisés comme antiviraux, parmi lesquels il convient de citer l'AZT, le DDC, le DDI, le D4T, le 3TC, le FddCIUrd, le carbovin, le PMEA, le PMPA sont tous dépourvus du groupement 3'-OH sur le carbone C3' du désoxyribose rendant incapable la chaîne naissante, ayant incorporé cet analogue nucléotidique terminateur de chaîne, d'échanger une liaison phosphodiester avec le nucléotide suivant. L'utilisation de tels analogues nucléotidiques terminateurs des chaînes ont été divulgués dans différentes publications scientifiques de l'art antérieur. Ainsi, les publications De Clercq (1997), Morris Jones *et al.* (1997), Patick *et al.* (1997), Debyser et De Clercq (1996), De Clercq et Balzarini (1995, Il Farmaco, 50 : 735-747) et Granier et Valantin (1998) évoquent l'utilisation d'antiviraux nucléosidiques / nucléotidiques terminateurs de chaîne comme agents mono- ou multithérapeutiques. Par ailleurs, la publication de Tong et al. (1997, Biochemistry 36 (19) : 5749-5757) décrit l'utilisation d'analogues nucléotidiques terminateurs de chaîne sous la forme triphosphate pour stabiliser le complexe formé entre la reverse-transcription et la molécule d'ADN. Tong *et al.* mentionnent uniquement l'utilisation du dUTP et de dITP pour la stabilisation du complexe formé afin d'étudier la résistance à la dissociation des complexes en fonction du nucléotide triphosphate utilisé et ne suggèrent à aucun moment une utilisation selon la présente invention. Egalement, un article de Mac Intosh et Haynes (1997) constitue une spéculation sur le rôle de l'enzyme d'UTPpyrophosphatase (dUTPase) virale qui catalyse la réaction de dephosphorylation du dUTP (triphosphate) en dUMP (monophosphate) comme moyen d'éviter que les dUTP cellulaires ne soient incorporés dans le génome viral ; en effet, les rétrovirus des primates dont le VIH fait partie n'ont pas de gènes codant une dUTPase. Les auteurs suggèrent que d'autres virus de primates codant la dUTPase supplé son absence chez le VIH ; les auteurs suggèrent en outre de combattre l'infection par le VIH en combattant ces virus pourvoyeurs d'UTPases. Enfin, l'art antérieur mentionnne une demande internationale de brevet WO 96 40 166 revendique l'utilisation d'analogues de nucléotide pour combattre l'hyperprolifération pathologique de cellules de la peau.

Par analogues de nucléotides on entend désigner tout nucléotides autres que les nucléotides dits "normaux" que sont l'acide adénylique ou déoxyadénylique, l'acide guanylique ou déoxyguanylique, l'acide cytidylique ou déoxycytidylique, l'acide thymidylique ou déoxythymidylique, l'acide uridylique. Les analogues de nucléotides peuvent être modifiés à n'importe quelle position des nucléotides ; les analogues de nucléotides peuvent être sous la forme de mono-, di- ou triphosphate, modifié ou non, pourvu que ces formes soient acceptées comme substrat par la reverse-transcriptase. Les analogues de nucléotides selon l'invention répondent plus généralement à deux critéres: (i) constituer un substrat de polymérisation par les reverse transcriptases ; (ii) ne pas bloquer la réaction de polymérisation nucléotidique au niveau du groupement situé sur le carbone C3' du 2'-désoxyribose de l'analogue nucléotidique incorporé; les molécules selon l'invention permettent l'addition d'au moins un nucléotide supplémentaire après incorporation dans la chaîne polynucléotidique naissante d'un analogue nucléotidique selon l'invention. Par la suite, on se référera pour simplifier à l'ensemble de ces molécules comme "analogues de nucléotides", sachant que la portée de l'invention ne se restreint pas aux analogues naturels et qu'elle inclut toute molécule capable de ces deux activités, qu'elle contienne une base purique ou pyrimidique ou non, et un oside pentose ou non.

Les analogues de nucléotides selon l'invention:
a) comprennent un motif 2'-désoxyribosyle comprenant en position 3' un groupement OH et en position 5' un groupement phosphate, le dit groupement phosphate étant choisi parmi le groupe constitué par les groupements mono-, di- et tri-phosphates, et
b) sont des substrats de la reverse transcriptase, ne terminent pas la réaction de la reverse transcriptase après leur incorporation dans la chaîne naissante, et leur groupement OH en position 3' est capable d'échanger des liaisons phosphodiester avec la chaîne naissante et le nucléotide suivant.

Ce qui caractérise les analogues nucléotidiques de l'invention est leur aptitude à poursuivre l'élongation de la chaîne nucléotidique en cours de synthèse, alors que les analogues nucléotidiques utilisés dans l'art antérieur ont une activité terminateur de chaîne.

Par « affection faisant intervenir la reverse transcriptase », on entend désigner (i) toutes les pathologies d'éthiologie virale causées par des virus possédant une enzyme ayant une activité reverse transcriptase ; l'ensemble de ces virus constitue le groupe des virus rétroïdes défini précédemment ; et (ii) tous les processus biologiques faisant appel à une étape de reverse-tanscription ; il peut s'agir des processus qui impliquent l'enzyme télomérase qui possède une activité reverse transcriptase, il peut s'agir des phénomènes de rétrotransposition d'éléments mobiles ou de séquences d'ADN (séquences LINE par exemple).

Les analogues de nucléotides utilisés dans la présente invention affectent les multiples fonctions biologiques virales : l'initiation, l'élongation et la terminaison de la reverse transcription, la fidélité et de là processivité de la reverse transcription, le contrôle de l'intégration du génome rétrotranscrit dans le génome cellulaire, l'expression et la réplication du génome rétrotranscrit et le cas échéant la production virale. Les effets biologiques de ces analogues nucléotidiques peuvent se manifester immédiatement ou ultérieurement, soit de la phase de reverse transcription en cours, soit au cours de l'une ou l'autre des activités biologiques prenant place lors du cycle de reverse transcription (expression génétique du génome rétrotranscrit, préparation du cycle de reverse transcription suivant ou ultérieur, cycle rétroviral le cas échéant) soit au cours d'un cycle de reverse transcriptase ultérieure. Les effets biologiques visés ci-dessus ne se manifestent que si les analogues de nucléotides ont été introduits lors de la reverse transcription du brin ADN(-) dont la matrice est le génome d'ARN viral, et/ou du brin ADN(+) dont la matrice est le brin ADN(-). Parmi les facteurs déterminant les effets biologiques, la fidélité et la processivité de la reverse transcription en présence des analogues de nucléotides possèdent une place prépondérante.

Les mécanismes et facteurs intervenant dans la fidélité et la processivité de la RT rétrovirale sont en effet exploités par le virus quand il utilise les nucléotides normaux, selon un dosage très précis, lui permettant de cheminer sur l'étroite ligne de crête entre deux précipices ; d'un côté, la progéniture virale risque d'avoir un taux d'infectivité insuffisant, par suite d'une fidélité et/ou d'une processivité insuffisante ; de l'autre coté, une grande stabilité génétique due à une fidélité et/ou une processivité élevée(s), donnera lieu à une stabilité antigénique suffisante pour une réponse immunitaire efficace de l'hôte. La présente invention consiste à utiliser des analogues de nucléotides pour prendre le contrôle de la fidélité et/ou de la processivité de la reverse transcriptase afin de faire basculer le virus dans l'un ou l'autre de ces précipices. Ainsi une augmentation de la fidélité et/ou de la processivité obtenue par l'utilisation d'analogues nucléotidiques selon l'invention a pour action d'allonger le délai entre l'antigénicité actuelle et une antigénicité nouvelle, ce qui a pour conséquence d'augmenter l'efficacité des lymphocytes circulants ; à l'inverse, une diminution de la fidélité et/ou de la processivité liée à l'utilisation d'analogues nucléotidiques selon l'invention a pour effet de réduire le titre des virus infectieux circulants.

Un des objets de la présente invention est l'utilisation d'analogues nucléotidiques permettant de contrôler à volonté la fidélité de la reverse transcription.

On entend désigner par fidélité, le taux d'erreur par base de la polymérase au cours de la synthèse d'une chaine polynucléotidique à partir du brin matrice. Par erreur, on entend : (i) la substitution d'une base qui correspond au remplacement d'une base complémentaire par une base non complémentaire (la base complémentaire d'une base matrice A est T (ADN) ou U(ARN), d'une base matrice G est C, d'une base matrice T (ADN) est A, d'une base matrice U (ARN) est A, d'une base matrice C est G) ; (ii) l'addition d'une ou plusieurs bases par rapport à la base matrice (insertion) ; (iii) l'omission d'une ou plusieurs bases par rapport à la base matrice (délétion). La fidélité est donc la somme des nucléotides substitués, délétés ou insérés par la reverse transcriptase (ou à l'occasion de remaniements génomiques), divisé par le nombre total de nucléotides du brin matrice reverse transcrit. La fidélité de la RT de HIV-1 par exemple est estimée à 10⁻² à 10⁻⁷ par nucléotide *in vitro* (Preston & Garvey, 1992), de 10⁻⁴ à 10⁻⁶ par nucléotide au cours d'un cycle de réplication *in vivo,* comparé à 10⁻⁹ à 10⁻¹¹ par nucléotide, habituel pour les ADN polymérases cellulaires. Ces moyennes ont une valeur limitée, dans la mesure où les mutations ne sont pas réparties au hasard, mais sont concentrées très préférentiellement en des "points chauds" mutationnels. La fidélité est susceptible d'être affectée par les mécanismes suivants :
1) Une modification de la probabilité d'appariement Watson-Crick (WC) avec la base matrice, ce qui a pour conséquence de provoquer des appariements non-Watson-Crick (i.e. des mésappariements) et ainsi de modifier le taux de mutation de substitution. Un certain nombre de facteurs sont susceptibles de favoriser les mésappariements au site actif ; on peut citer : (i) les facteurs physico-chimiques au site actif influant sur la tautomérisation ou l'ionisation des nucléotides ; (ii) les facteurs stériques au site actif responsables d'une présentation du nucléotide triphosphate à la base matrice dans une disposition spatiale permettant l'un des multiples appariements non Watson-Crick, dont en particulier l'appariement Wobble ; (iii) les facteurs cinétiques de la polymérisation, telle la concentration de nucléotide triphosphate au site actif, qui affectent la probabilité d'appariement Watson-Crick ou de mésappariement.
2) Une omission de complémenter une ou plusieurs base(s) de la matrice, ce qui a pour conséquence de modifier le taux de mutation de délétion ;
3) Une addition de une ou plusieurs base(s) sans matrice, ce qui a pour conséquence de modifier le taux de mutation d'insertion ; un certain nombre de facteurs sont susceptibles de favoriser les mutations de délétion et d'insertion au site actif : (i) les "glissements", rendus possibles par la présence de courtes séquences identiques voisines (Bebenek K. & Kunkel, T.A. 1990 Proc. Natl. Acad. Sci. USA 4946-50), les glissements sont souvent initiés par un appariement non-Watson-Crick ; (ii) une distance et/ou une présentation spatiale inappropriée(s) de l'extrémité 3'0H du brin naissant par rapport au nucléotide triphosphate (au site actif) et/ou la base matrice, pouvant conduire à une délétion d'une base si la distance est trop grande ou une insertion d'une base si elle est trop courte ; distance et présentation spatiale sont contrôlées entre autres par la conformation précise de la double hélice d'environ 20 paires de bases couverte par la RT, conformation qui dépend de la séquence et/ou de la présence d'analogues de nucléotides.
4) les réarrangements et/ou les recombinaisons génomiques.

Les analogues de nucléotides permettent de prendre le contrôle de la fidélité de la reverse transcription selon des mécanismes exposés ci-dessus. Les conditions de la prise de contrôle de la fidélité dépendent de la nature des modifications de l'analogue de nucléotides. C'est donc un des objets de la présente invention d'utiliser des analogues nucléotidiques pour introduire des mutations contrôlées des bases telles que des mutations de substitution, des mutations de délétion, des mutations d'insertion.

Un autre aspect de la présente invention concerne l'utilisation d'analogues nucléotidiques permettant de contrôler à volonté la processivité de la reverse transcriptase.

La processivité se définit comme le nombre de nucléotides incorporés dans le brin néosynthétisé avant que l'enzyme ne se dissocie du complexe réactionnel, que la dissociation soit ou non précédée d'une pause de l'élongation de la reverse transcription. Cette dissociation est "spontanée", c'est-à-dire qu'elle n'est pas "forcée" par le bloquage en 3' du nucléotide, qui interdit toute élongation.

Il existe différents mécanismes susceptibles d'affecter la processivité de la reverse transcriptase ayant en commun d'empêcher l'addition du nucléoside triphosphate à la chaîne naissante, c'est-à-dire la réaction conduisant au lien phosphoester entre le phosphate en 5' du nucléotide entrant et le groupement OH en 3' du nucléotide terminal de la chaîne naissante. On peut citer les mécanismes suivants :
1- mécanisme stérique :
   a)- le nucléotide triphosphate au site actif est trop éloigné ou se présente mal stériquement par rapport au 3'OH de la chaîne naissante, ce dernier occupant une position normale dans la RT, le motif du désordre étant, inhérent au site actif et/ou au nucléotide triphosphate, apparié ou non selon un appariement Watsori-Crick ou non Watson-Crick ;
   b)- le nucléotide triphosphate au site actif est trop éloigné ou se présente mal stériquement par rapport au 3'OH de la chaîne naissante, ce dernier occupant une position anormale dans la RT, du fait par exemple d'une conformation anormale de la double-hélice engagée dans la RT ;
   c)- le jeu qu'autorise la RT au site actif éloigne le nucléotide triphosphate du groupement 3'0H, tous deux étant positionnés correctement par ailleurs.

   Ce mécanisme stérique est possible du fait de la flexibilité des RT ; en effet, la distance entre le phosphate gamma du nucléotide et l'extrémité 3'0H du brin naissant n'est pas figée. De plus, le domaine qui "couvre" la double hélice (sortante) présente un certain degré de liberté par rapport au site catalytique et par rapport au domaine en contact avec le brin matrice entrant dans l'enzyme (par exemple HIV-1); enfin, pour les RT dont le mécanisme catalytique est connu (par exemple HIV-1), un dysfonctionnement de la translocation du nucléotide à incorporer du site d'entrée vers le site catalytique aboutissant à empécher l'addition du nucléotide peut également affecter la processivité. Les analogues de nucléotides peuvent agir sur la processivité mettant en jeu une distance et/ou une présentation spatiale inappropriée(s) de l'extrémité 3'OH du brin naissant par rapport au nucléotide modifié au site actif et/ou la base matrice, ce qui peut conduire à une délétion ou une insertion d'une base, selon que la modification réduit ou augmente la distance et/ou la présentation spatiale, de façon à ignorer une base matrice ou insérer une base dépourvue de base matrice. Distance et présentation spatiale sont contrôlées entre autres par la conformation précise de la double hélice de environ 20 paires de bases couverte par la RT, conformation qui dépend de la séquence et/ou de la présence de analogues de nucléotides, ainsi que par la rigidité ou la flexibilité du nucléotide triphosphate modifié qui se situe au site actif.
2- mécanisme cinétique :
   Une pause prolongée, due par exemple à une concentration trop faible de nucléotide triphosphate, conduit l'enzyme à quitter la chaîne naissante. Les RT sont, en général, bien moins processives que les ADN- ou ARN-polymérases et ont une tendance spontanée à se dissocier du complexe de reverse-transcripion. Plus la durée d'une pause de la reverse transcriptase (pour un motif quelconque) est longue, plus la dissociation est probable et donc plus la processivité au site de pause diminue.

Les analogues de nucléotides permettent de prendre le contrôle de la processivité selon des mécanismes semblables à ceux exposés ci-dessus pour les nucléotides normaux, mais avec une démultiplication contrôlée des effets de l'un et/ou l'autre des divers facteurs mentionnés, selon la modification présente sur les analogues de nucléotides.

La présente invention concerne l'utilisation d'analogues nucléotidiques caractérisés en ce que lesdits analogues nucléotidiques
- diminuent de façon contrôler la fidélité de la reverse transcription et/ou abaissent de façon contrôlée la processivité de la reverse transcriptase et/ou en diminuent de façon contrôlée le temps de pause de la reverse transcriptase ;
- augmentent de façon contrôlée la fidélité de la reverse transcription et/ou augmente de façon contrôlée la processivité de la reverse transcriptase et/ou allonge de façon contrôlée le temps de pause de la reverse transcriptase.

Parmi les affections faisant intervenir la reverse-transcriptase, il y a celles provoquées par les reverse-transcriptases des virus rétroïdes. Parmi les affections provoquées par les virus rétroïdes qui peuvent être traitées par les analogues nucléotidiques utilisés selon la présente invention il faut citer, les virus rétroïdes qui infectent l'homme et/ou les animaux et/ou les végétaux notamment les virus rétroïdes qui infectent les cellules humaines et/ou animales, et qui appartiennent plus particulièrement au groupe des lentivirus, des oncovirus à ARN, des spumavirus et des hépadnavirus. Selon un mode préféré de réalisation de l'invention, le lentivirus est choisi parmi le groupe composé du virus de l'immunodéficience humaine (HIV), et de préférence du virus de l'immunodéficience humaine de type 1 (HIV-1) et les virus de la leucémie des cellules T humaines (HTLV).

Selon un autre mode préféré de réalisation de l'invention, l'utilisation de l'analogues nucléotidiques selon la présente invention est caractérisée en ce que l'hépadnavirus est le virus de l'hépatite B (HBV). L'HBV est un virus à ADN mais dont le génôme passe sous une forme ARN lors de sa réplication ; il y a ensuite reverse transcription pour donner naissance à l'ADN génomique.

La présente invention concerne l'utilisation des analogues de nucléotides tels que décrits précédemment et tels que lorsqu'ils sont incorporés par la reverse-transcriptase dans la chaîne polynucléotidique, ils introduisent des mésappariements.

On entend désigner par mésappariements, tous appariements qui ne respectent pas très précisément les appariements Watson-Crick. Dans la présente invention, on entend désigner par appariement Watson-Crick, désigné WC ci-après, l'appariement défini par des liaisons hydrogène suivantes (schéma 1) :
*a) pour les appariements adénine (A)-uracyle (U) ou thymine (T)*
   1) N6 de A donneur de H6 à l'accepteur 04 de U ou T
   2) N 1 de A accepteur de H3 du donneur N3 de U ou T
*b) pour les appariements guanine (G)-cytidine (C)*
   1) 06 de G accepteur de H4 du donneur N4 de C
   2) N1 de G donneur de H1 à l'accepteur N3 de C
   3) N2 de G donneur de H2 à l'accepteur 02 de C

Tous appariements qui ne respectent pas très précisément ces liaisons hydrogène entre ces 5 nucléotides, soit qu'ils ne les respectent que partiellement, soit qu'ils les respectent mais avec d'autres liaisons H en sus, soit qu'ils ne les impliquent pas, soit que l'un et/ou l'autre des partenaires de la paire ne font pas partie de ces 5 nucléotides (exemple nucléotide avec purine dont le N7 a été remplacé par C7), définissent l'ensemble des appariements désignés dans la présente invention comme "mésappariements" . Ainsi, les exemples (schéma 2) illustrent de manière non limitative certains mésappariements impliquant les 5 nucléotides normaux:
1) *l'appariement A-T* "reverse *WC"*
   N6 de A donneur de H6 à l'accepteur 02 de T
   N 1 de A accepteur de H3 du donneur N3 de T
*2) l'appariement A-T "Hoogsteen*"
   N6 de A donneur de H6 à l'accepteur 04 de T
   N 7 de A accepteur de H3 du donneur N3 de U ou T
*3) l'appariement A-T* "reverse *Hoogsteen"*
   N6 de A donneur de H6 à l'accepteur 02 de T
   N 7 de A accepteur de H3 du donneur N3 de T
*4) l'appariement "wobble" G ou l-U ou T*
   N1 de G ou I donneur de H1 à l'accepteur 02 de U ou T
   06 de G ou I accepteur de H3 du donneur N3 de U ou T

Il faut également mentionner que les bases normales peuvent se présenter sous forme de tautomères, keto ou énol pour G et T, amino ou imino pour A et C ; les fréquences des formes énol et imino sont faibles mais non nuls (de l'ordre de 10⁻² à 10⁻⁴). Ces tautomères ont des caractéristiques de donneurs et accepteurs de protons propres (schéma 3) et peuvent donner lieu à des appariements tels T-G(énol), T(énol)-G, A(imino)-C ou A-C(imino) (schéma 4).

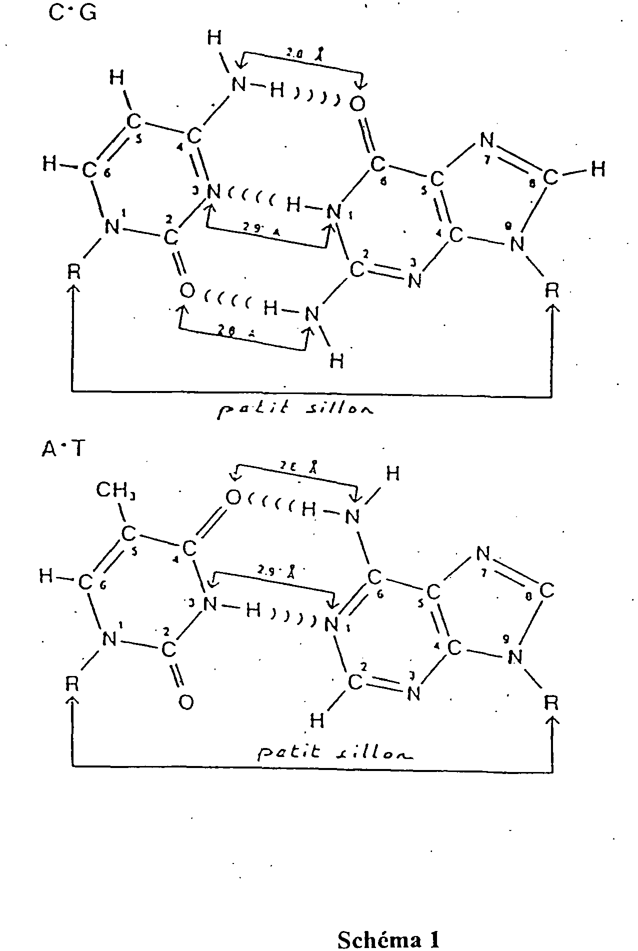

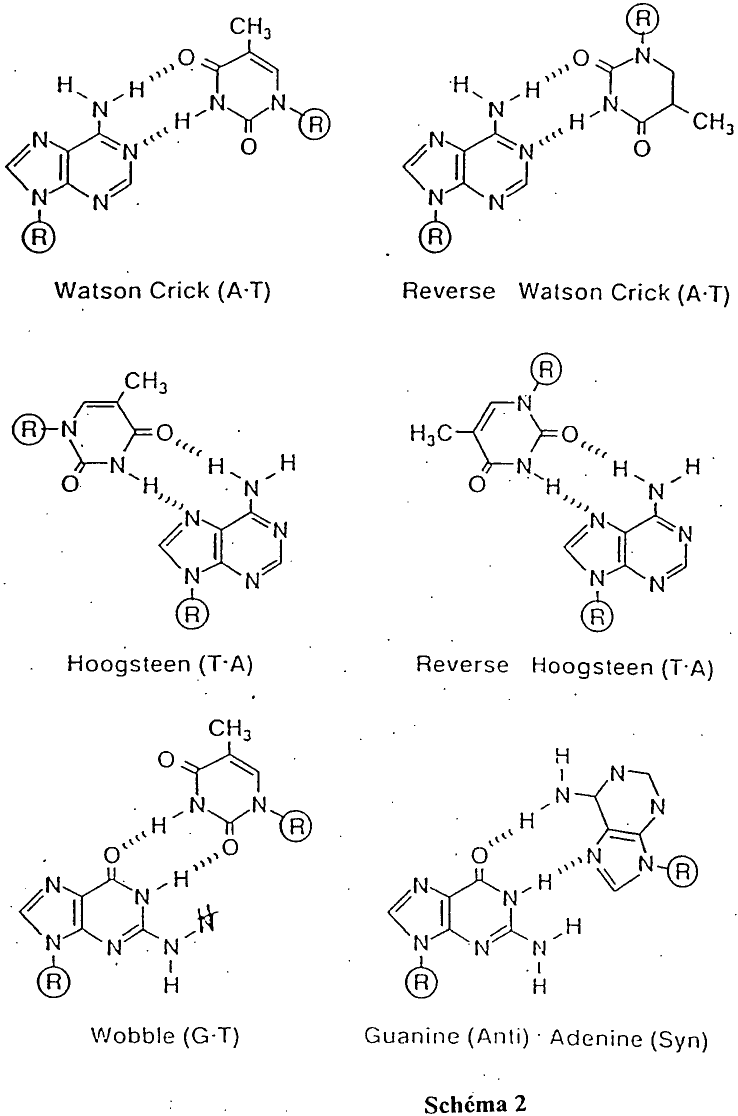

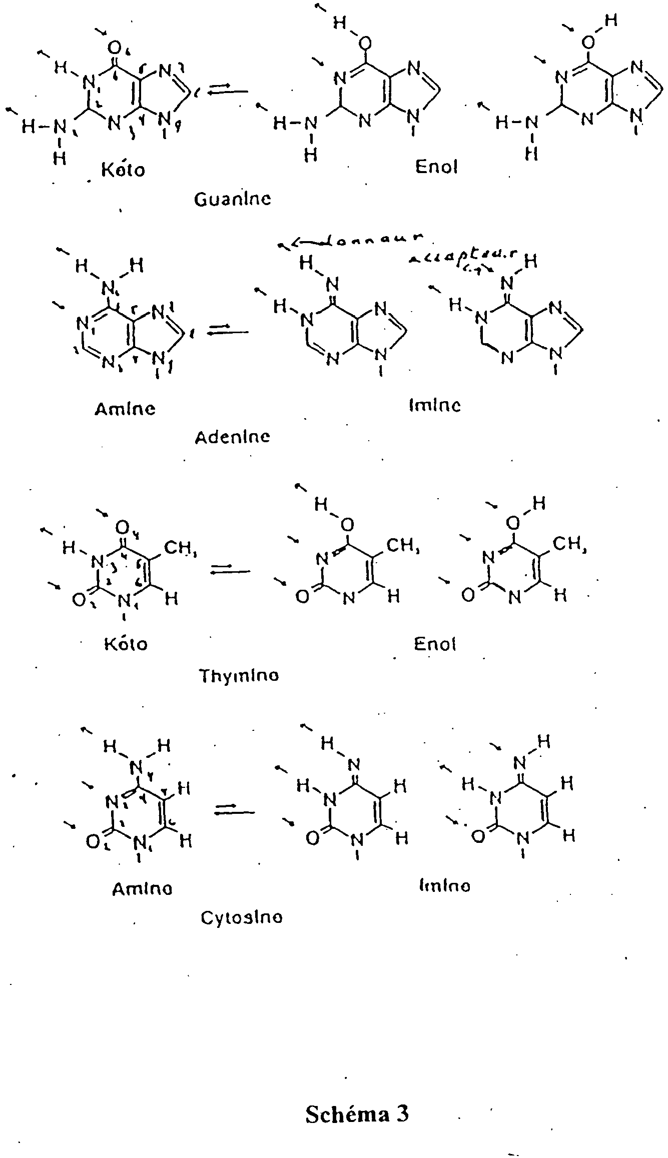

Enfin, les bases normales sont suceptibles de s'ioniser. Ainsi A s'ionise facilement en A(+) à pH acide, C en C(+) aussi, G en G(-) et T en T(-). Se forment ainsi les paires C-A(+), T(-)-G, C(+)-G, A(+)-G(-) (schéma 5).

Toutes ces possibilités d'appariement Watson-Crick et de mésappariements décrites ci-dessus pour les nucléotides de bases "normales" (ou conventionnelles) (A,T,U,G,C) sont concevables également avec ceux des analogues de nucléotides de la présente invention, avec des probabilités (barrière énergétique à surmonter) et des stabilités (profondeur du puits de potentiel) d'appariement variables (en plus ou en moins) par rapport aux nucléotides normaux, ainsi que des qualités diverses de substrats pour l'enzyme.

La présente invention concerne notamment l'utilisation d'analogues nucléotidiques incorporés par la reverse-transcriptase dans la chaine polynucléotidique et qui s'apparient selon un appariement de type Wobble. Selon la présente invention, on entend désigner par appariement de type Wobble des appariements violant la règle des appariements de type Watson-Crick tels que définis précédemment. Dans les appartements de type Wobble, deux bases sont liées par des liaisons hydrogènes différentes de celles de la paire de bases Watson-Crick, résultant ainsi de relations spatiales différentes. La liaison glycosidique est de manière caractéristique en configuration ANTI. La position d'une des bases par rapport à sa base complémentaire est décalée (glissée) dans le plan plat des noyaux aromatiques et des liaisons hydrogènes (Saenger, 1984).

La présente invention concerne également l'utilisation d'analogues nucléotidiques incorporés par la reverse-transcriptase dans la chaine polynucléotidique et qui s'apparient selon un appariement de type ANTI-SYN. Selon la présente invention, on entend désigner par appariement de type ANTI-SYN, des appariements violant la règle des appariements Watson-Crick. Dans les appariements ANTI-SYN la liaison glycosidique est de manière caractéristique en configuration ANTI. La paire ANTI-SYN se forme entre deux purines dans lesquelles une purine est dans la forme ANTI et l'autre dans la forme inhabituelle SYN.

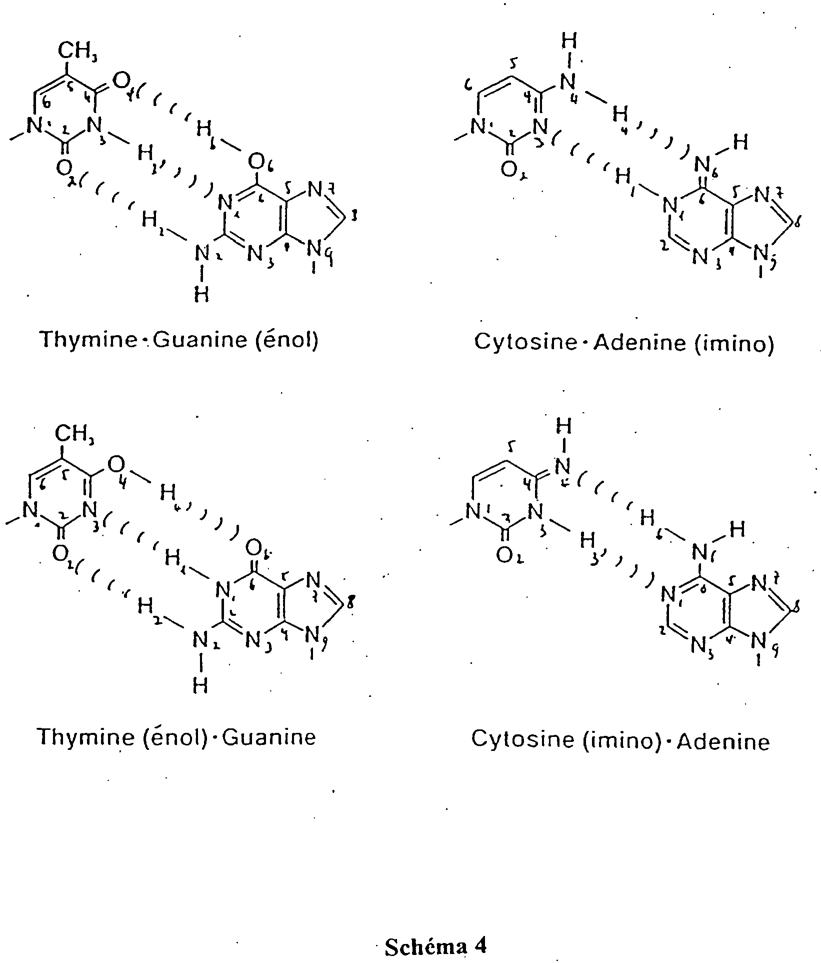

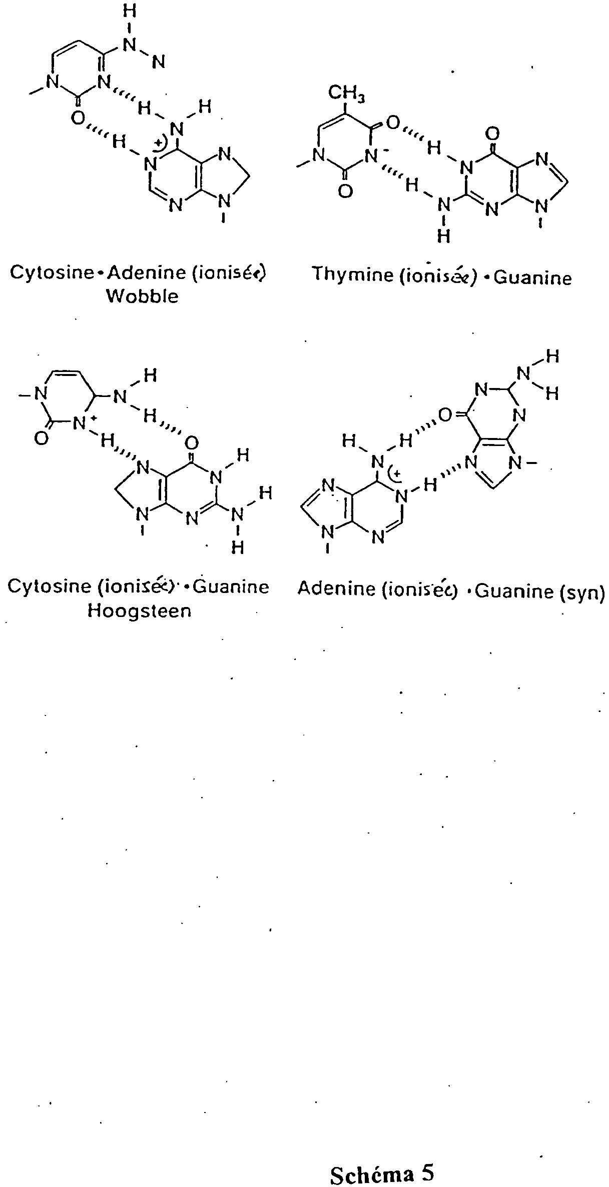

La présente invention concerne également l'utilisation d'analogues nucléotidiques incorporés par la reverse-transcriptase dans la chaine polynucléotidique et qui s'apparient selon un appariement de type Hoogsteen et/ou de type reverse Hoogsteen. Selon la présente invention, on entend désigner par appariement de type Hoogsteen et de type reverse Hoogsteen des appariements violant la règle des appariements de type Watson-Crick. Dans la paire Hoogsteen la distance entre les atomes C_{1'} et C_{1'} est de 8.645 Å; elle est donc réduite d'environ 2 Å comparée à la paire de bases Watson-Crick; Les angles entre la ligne C_{1'}...C_{1'} et les liaisons glycosyliques C_{1'} - N diffèrent de plus de 10° entre les bases purine et pyrimidine. Dans une paire de base Hoogsteen, la pyrimidine utilise sa surface Watson-Crick pour s'apparier avec le côté N1, C6, N7 de la base purine. Enfin, une rotation de 180° de la pyrimidine résulte dans la formation d'une paire de base reverse Hoogsteen (Saenger, 1984).

La présente invention concerne également l'utilisation d'analogues nucléotidiques incorporés par la reverse transcriptase dans la chaîne polynucléotidique et qui s'apparient selon un appariement de type reverse Watson-Crick. Selon la présente invention, on entend désigner par appariement de type reverse Watson-Crick, des appariements violant la règle des appariements de type Watson-crick, les paires de base reverse Watson-crick se forment lorsqu'un nucléotide effectue une rotation de 180° par rapport au nucléotide complémentaire. Dans ce cas les liaisons glycosidiques (et le squelette sucre phosphate) sont en trans plutôt qu'en orientation cis. A cause de la symétrie dans les liaisons hydrogènes potentielles de la thymine aux portions C2-N3-C4, la base T peut tourner autour de l'axe N3-C6 pour former une paire de base A-T selon un appariement reverse Watson-Crick (Saenger, 1984).

Un grand nombre d'analogues nucléotidiques sont utilisables dans là mise en oeuvre de la présente invention, ces analogues nucléotidiques sont notamment choisis parmi l'ensemble des nucléotides modifiés naturels, et plus particulièrement parmi les nucléotides modifiés présents dans les ARN procaryotes ou eucaryotes (voir tableau 1), mais la présente invention ne se restreint pas à ces seuls nucléotides modifiés naturels. Une liste non exhaustive de ces nucléotides a été publiée par Limbach et al (1995) et Motorin et Grosjean (1998). Selon la présente invention, les analogues nucléotidiques utilisés sont choisis de préférence parmi l'ensemble des nucléotides modifiés des ARN de transfert (ARNt) procaryotes ou eucaryotes. Les ARN de transfert (ARNt) contiennent plus de 100 espèces de nucléotides modifiés (voir tableau 1) dont les structures ont été établies. Selon un autre aspect de la présente invention, les analogues nucléotidiques utilisés sont choisis parmi le dUTP, le dITP, le C7dGTP et le C7dATP.

**Tableau 1 : nucléosides modifiés présents dans les ARN**

| **NOM** | **SYMBOLE** |
|---|---|
| **Dérivés d'uridine** | |
| Pseudouridine (P) | Ψ |
| 2'-O-méthylpseudouridine (Z) | Ψm |
| 1-méthylpseudouridine (I) | m¹Ψ |
| 3-méthylpseudouridine | m³Ψ |
| 1-méthyl-3-(3-amino-3-carboxypropyl)pseudouridine | m¹acp³Ψ |
| | |

| **Dérivés de cytidine** | |
|---|---|
| 2'-O-méthylcytidine (B) | Cm |
| N⁴-méthylcytidine | m⁴C |
| N⁴,2'-O-diméthylcytidine | m⁴Cm |
| N⁴-acétylcytidine (M) | ac⁴C |
| N⁴-acéthyl-2'-O-méthylcytidine | ac⁴Cm |
| 5-méthylcytidine | m⁵C |
| 5,2'-O-diméthylcytidine | m⁵Cm |
| 5-hydroxyméthylcytidine | hm⁵C |
| 5-formylcytidine | *f*⁵C |
| 2'-O-méthyl-5-formylcytidine | f⁵Cm |
| 3-méthylcytidine | m³C |
| 2-thiocytidine | s²C |
| lysidine | k^{2C} |
| 2'-O-méthyluridine (J) | Um |
| 2-thiouridine | s²U |
| 2-thio-2'-O-méthyluridine | s²Um |
| 3-méthyluridine | m³U |
| 3,2'-O-diméthyluridine | m³Um |
| 3-(3-amino-3-carboxypropyl)uridine (X) | acp³U |
| 4-thiouridine | s⁴U |
| Ribosylthymine (T) | m⁵U |
| 5,2'-O-diméthyluridine | m⁵Um |
| 5-méthyl-2-thiouridine (F) | m⁵s²U |
| 5-hydroxyuridine | ho⁵U |
| 5-méthoxyuridine | mo⁵U |
| uridine 5-oxyacetic acid (V) | cmo⁵U |
| uridine 5-oxyacetic acid méthyl ester | mcmo⁵U |
| 5-carboxyméthyluridine | cm⁵U |
| 5-méthoxycarbonylméthyluridine | mcm⁵U |
| 5-méthoxycarbonylméthyl-2'-O-méthyluridine | mcm⁵Um |
| 5-méthoxycarbonylméthyl-2-thiouridine | mcm⁵s²U |
| 5-carbamoylméthyluridine | ncm⁵U |
| 5-carbamoylméthyl-2'-O-méthyluridine | ncm⁵Um |
| 5-(carboxyhydroxyméthyl)uridine | chm⁵U |
| 5-(carboxyhydroxyméthyl)uridineméthyl ester | mchm⁵U |
| 5-aminométhyl-2-thiouridine | nm⁵s²U |
| 5-méthylaminométhyluridine | mnm⁵U |
| 5-méthylaminométhyl-2-thiouridine (S) | mnm⁵s²U |
| 5-méthylaminométhyl-2-sélénouridine | mnm⁵se²U |
| 5-carboxyméthylaminométhyluridine | cmnm⁵U |
| 5-carboxyméthylaminométhyl-2'-O-méthyluridine | cmnm⁵Um |
| 5-carboxyméthylaminométhyl-2-thiouridine | cmnm⁵s²U |
| Dihydrouridine (D) | D |
| Dihydroribosylthymine | m⁵D |
| | |

| **Dérivés d'adénosine** | |
|---|---|
| 2'-O-méthyladénosine | Am |
| 2-méthyladénosine | m²A |
| N⁶-méthyladénosine | m⁶A |
| N⁶,N⁶-diméthyladénosine | m⁶₂A |
| N⁶,2^{.}-O-diméthyladénosine | m⁶Am |
| N⁶,N⁶,2-O-triméthyladénosine | m⁶₂Am |
| 2-Méthylthio-N⁶-méthyladénosine | ms²m⁶A |
| N⁶-isopentényladénosine | i⁶A |
| 2-méthylthio-N⁶-isopentényladénosine | ms²i⁶A |
| N⁶-(cis-hydroxyisopentényl)adénosine | io⁶A |
| 2-méthylthio-N⁶-(cis-hydroxyisopentényl)-adénosine | ms²io⁶A |
| N⁶-glycinylcarbamoyladénosine | g⁶A |
| N⁶-thréonylcarbamoyladénosine | t⁶A |
| N⁶-méthyl-N⁶-thréonylcarbamoyladénosine | m⁶t⁶A |
| 2-méthylthio-N⁶-thréonylcarbamoyl-adénosine | ms²t⁶A |
| N⁶-hydroxynorvalylcarbamoyladénosine | hn⁶A |
| 2-méthylthio-N⁶-hydroxynorvalylcarbamoyl-adénosine | ms²hn⁶A |
| 2'-O-ribosyladénosine (phosphate) ' | Ar(p) |
| 1-méthyladénosine | m¹A |

| **Dérivés d'inosine** | |
|---|---|
| inosine (I) | I |
| 2'-O-méthylinosine | Im |
| (O) | m^{I}I |
| 1,2'-O-diméthylinosine | m^{I}Im |
| | |

| **Dérivés de guanosine** | |
|---|---|
| 2'-O-méthylguanosine | Gm |
| 1-méthylguanosine (K) | m¹G |
| N²-méthylguanosine (L) | m²G |
| N²,N²-diméthylguanosine (R) | m²₂G |
| N²,2'-O-diméthylguanosine | m²Gm |
| N²,N²,2-O-triméthylguanosine (I) | m²₂Gm |
| 2'-O-ribosylguanosine (phosphate) | Gr(p) |
| | |

| **Dérivés de m⁷-Guanosine** | |
|---|---|
| 7-méthylguanosine (7) | m⁷G |
| N²,7-diméthylguanosine | m^{2,7}G |
| N²,N²,7-triméthylguanosine | m^{2,2,7}G |
| | |

| **Dérivés de wyosine** | |
|---|---|
| Wyosine | imG |
| Méthylwyosine | mimG |
| Hydroxywybutosine sous-modifié | OhyW |
| Wybutosine (Y) | yW |
| Hydroxywybutosine | OhyW |
| Péroxywybutosine (W) | o₂yW |
| | |

| **Dérivés de 7-déazaguanosine** | |
|---|---|
| queuosine (Q) | Q |
| Epoxyqueuosine | oQ |
| galactosyl-queuosine | galQ |
| mannosyl-queuosine | manQ |
| 7-cyano-7-déazaguanosine | preQₒ |
| Archaeosine (autre appélation 7-formamidino-7-déazaguanosine) | gQ (G⁺) |
| 7-aminométhyl-7-déazaguanosine | preQ₁ |

Un autre aspect de la présente invention est l'utilisation d'analogues nucléotidiques caractérisés en ce qu'ils ne sont pas présents naturellement dans les cellules vivantes; selon la présente invention, les analogues nucléotidiques modifiés obtenus par voie de synthèse possèdent de préférence les bases modifiés **6H, 8H-3, 4-dihydropyrimido [4,5c] [1,2] oxazin-7-one (P) et N⁶-méthoxy-2,6-diaminopurine (K)** et (Hill et al. (1998) Proc. Natl. Acad. Sci. USA 95: 4258-4263).

**De préférence,** les analogues nucléotidiques utilisés dans la présente invention sont **des 2'-déoxyribose sous la forme triphosphate et présentent un groupement hydroxyl (-OH) sur le carbone C3' du déoxyribose.** Selon l'invention, les analogues nucléotidiques tels que définis ci-dessus peuvent comporter des groupements protecteurs des groupes fonctionnels afin de préserver les bases modifiées lors de leur métabolisme. Les analogues de nucléotides peuvent être protégés sous la forme de pronucléotides **bis-(S-acyl-2-thioéthyl) (BIS-SATE)** (Benzaria et al., 1996 ; Lefebvre et al., 1995).

Un des avantages de la présente invention est que les analogues nucléotidiques naturels peuvent s'obtenir facilement et en grande quantité à partir de culture de microorganismes ; de telles cultures sont économiques et peuvent facilement s'effectuer à grande échelle (Gehrke et Kuo, 1985).

La présente invention concerne également une composition pharmaceutique caractérisée en ce qu'elle contient des analogues nucléotidiques tels que définis ci-dessus et un véhicule pharmaceutiquement acceptable.

Dans un mode particulier de réalisation de l'invention, la composition pharmaceutique de la présente invention est caractérisée en ce qu'elle contient un mélange d'au minimum un analogue nucléotidique et d'au miminum un agent antirétroviral choisi parmi le groupe composé des inhibiteurs de la reverse transcriptase nucléotidiques et non nucléotidiques et des antiprotéases virales comme produit de combinaison pour une utilisation simultanée, séparée ou étalée dans le temps en thérapie antivirale; l'inhibiteur de la reverse transcriptase est de préférence choisi parmi le 3'-azido-3'déoxythymidiné (AZT), le 2',3'-didéoxyinosine (ddI), le 2',3'-didéoxycytidine (ddC), le (-)2',3'-didéoxy-3'-théacytidine (3TC), le 2',3'-didéhydro-2',3'didéoxythymidine (d4T) et le (-)2'-déoxy-5-fluoro-3'-théacytidine (FTC), le TIBO, le HEPT, le TSAO, l'α-APA, la névirapine, le BAHP, l'acide phosphonoformique (PFA) ; l'antiprotéase virale est choisie parmi l'indinavir et le saquinavir.

La possibilité de prendre le contrôle des caractéristiques génétiques du matériel rétro-transcrit permet de manipuler les progénitures virales dans les cycles de réplication ultérieurs du virus. Cette activité différée laisse une large latitude de manoeuvre à l'initiative du praticien, qui peut choisir les analogues de nucléotides et les doses cadrant avec la stratégie particulière adaptée à un patient particulier.

Cette latitude n'existe pas pour les antiviraux immédiats. Pour ces derniers en effet, ou bien le virus ne peut se soustraire à l'action de l'antiviral, et celui-ci élimine le virus, ou bien le virus trouve une parade et l'antiviral devient inefficace. Le fort taux de mutation qui caractérise la reverse-transcription favorise grandement cette dernière alternative.

Le fait de laisser la reverse-transcription se poursuivre évite deux écueils auxquels se heurtent les antiviraux immédiats. D'abord, le virus est soumis par l'antiviral immédiat à une pression de sélection. La faible fidélité de sa reverse-transcriptase permet au virus d'échapper rapidement à cet antiviral, d'où l'émergeance rapide de mutants de résistance. En cas de multithérapies par association d'antiviraux immédiats, cette émergeance est plus lente mais finit le plus souvent par survenir. Ensuite, l'antiviral ou ses associations réduisent pour un temps la charge virale (souvent en dessous du seuil de détection) avant l'émergeance de la variété virale résistant. Cette dernière, débarrassée de compétiteurs, va alors pouvoir envahir l'organisme, souvent de façon foudroyante. A l'inverse, les analogues nucléotidiques de l'invention évitent ces deux écueils. **D'abord, l'absence de bloquage de la reverse-transcription ote toute** pression de sélection durant le cycle de reproduction virale en cours. L'action sur le virus, en l'occurrence celle résultant de l'altération génétique du matériel rétrotranscrit, programmée à l'initiative du praticien, est différée c'est-à-dire ne se manifeste pour l'essentiel que sur les progénitures virales futures. Ensuite, les analogues nucléotidiques selon l'invention étant essentiellement dépourvus d'activité antivirale immédiate, la charge virale globale reste momentanément presque constante. La compétition entre quasi-espèces virales sera peu affectée. ce qui limitera l'expression d'une quasi-espèce particulière.

Des caractéristiques et avantages de la présente invention seront mieux mis en évidence à la lecture des exemples suivants.

### Exemple 1 : Choix de la séquence

Modrow et al. en 1987 ont montré que les mutations les plus fréquentes sont localisées au sein du gène *env* du génome de HIV, sur 5 sites hypervariables (V1 à V5). La grande variabilité génétique dans le gène *env* pourrait être la raison principale de la difficulté de produire un vaccin contre la coque virale, ce qui rend d'autant plus intéressant l'étude du mécanisme de ces mutations. La *synthèse in vitro* par la reverse transcriptase de HIV du brin (+) (c'est-à-dire ayant pour matrice le brin ADN (-) issu de la transcription inverse du génome viral) montre que les mutations au site de pause majeure (V1 sur une suite de six A (bases 480 à 485 du gène *env*)) sont pour les trois-quarts des substitutions de bases (AT→GC et GC→AT), le reste étant des glissements (Ji et al., 1994).

Ce travail consiste à étudier la région hypervariable V1, dans sa version ADN. Dans ce but, une séquence d'une centaine de bases de la séquence virale *env* entourant les six adénines (base 480 à 485 du gène *env*) a été prolongée aux deux extrémités par deux segments de 17 bases, dans laquelle l'une des 4 bases est absente dans le but de pouvoir initier la réaction de polymérisation avec trois des 4 dNTPs seulement).

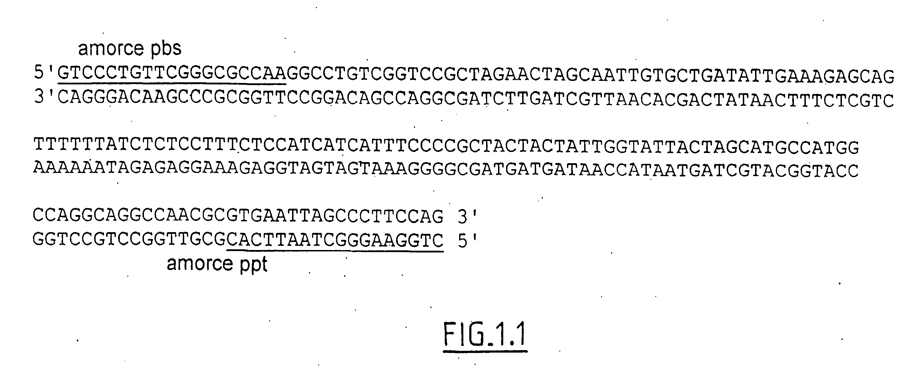

Les séquences " amorces " naturelles de HIV sont accrochées aux deux extrémités du fragment du gène, à savoir à l'extrémité 3' la séquence de tARN (lys3) (séquence complémentaire au primer binding site, pbs) et à l'extrémité 5' la séquence homopurine (polypurine tract, ppt). Cette construction est montrée Figure 1.1.

Ainsi la transcription inverse à partir de l'amorce pbs utilisera comme matrice le brin codant du gène *env,* qui sert de matrice à la synthèse du brin ADN-: il sera désigné ci-après par *env-.* De même, l'élongation à partir de l'amorce ppt aura comme matrice le brin ADN- et produira le brin ADN+. Cette matrice sera donc désignée *env+.* La raison qui nous a incité à inclure pbs et ppt dans notre construction était double: mimer les deux amorces naturelles, et explorer l'effet d'amorces dont les contraintes en composition purines/pyrimidines sont remarquables et donnent probablement lieux à des particularités conformationnelles et/ou, thermodynamiques.

La méthode d'étude consiste alors à prélever le plasmide simple-brin contenant la séquence *env -* (ou *env* +), à hybrider l'amorce pbs (ou ppt), d'initier la transcription inverse dans les conditions choisies. L'élongation sur les deux brins complémentaires montrera l'effet des deux amorces ainsi que des similitudes ou des différences sur des sites en regard.

Ces matrices ont été utilisées pour des études parallèles de la reverse transcriptase de HIV-1 et du fragment de Klenow de l'ADN polymérase ADN-dépendante d*'E.coli* (KF). De nombreuses études ont porté sur la fidélité et la processivité du fragment de Klenow (Abbotts et Wilson, 1988 ; Lecomte et Ninio, 1987,1988 ; Papanicolaou et Ripley, 1989 et 1991).

Des études de terminaison et de fidélité de la transcription inverse ont été effectuées.

### Exemple 2 : Identification des sites de terminaison

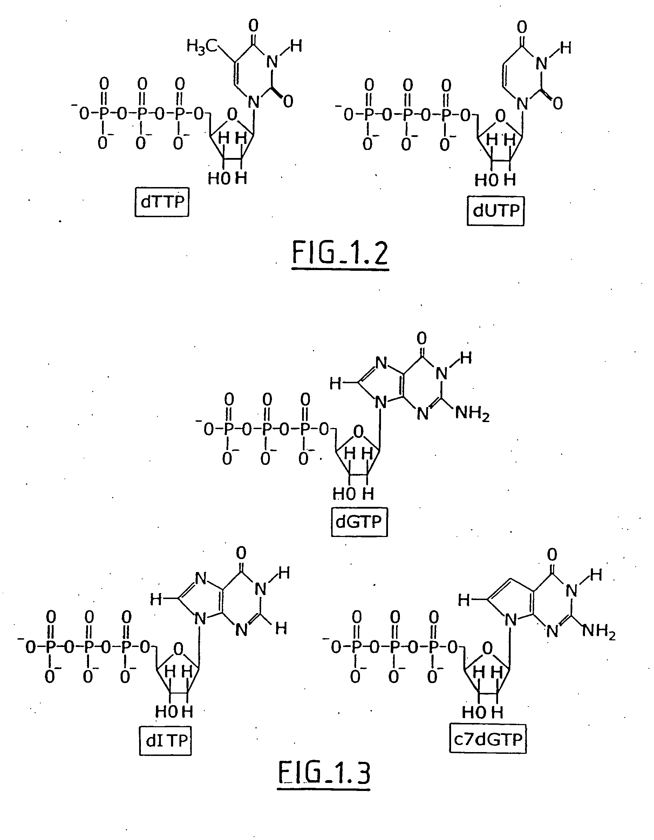

Les sites de terminaison et les taux de terminaison à chaque site ont été étudiés en présence de 4 dNTPs naturels présents en excès, ou en présence de trois de ces dNTPs naturels, le quatrième étant remplacé par un analogue: le dUTP en remplacement du dTTP, le dITP et le c7dGTP en remplacement du dGTP, enfin le c7dATP à la place du dATP (Figures 1.2 et 1.3). Ces analogues affectent la formation des liaisons hydrogène dans les deux sillons de la double hélice.

L'objectif a été d'évaluer également le rôle d'analogues nucléotidiques sur le taux de terminaison de la reverse transcriptàse de HIV-1 et du fragment de Klenow et de chercher les agents responsables de la terminaison de la synthèse avec les dNTPs "normaux" 4 analogues ont été retenus :
- le dUTP, analogue de dTTP, n'expose pas de CH₃ dans le grand sillon comme le fait le groupement méthyle en position C5 de dTTP. dUTP peut s'apparier avec A dans une conformation de type Watson-Crick, mais également avec G dans une conformation de type "Wobble".
- Le dITP diffère de dGTP par l'absence du groupement NH₂ en position C2, ce qui conduit à l'absence d'un donneur dans le petit sillon par rapport à dGTP. L'appariement C:I par rapport à C:G conduit à la présence d'un accepteur (l'oxygène de C) et l'absence d'un donneur (NH₂ de I) dans le petit sillon.
- Le c7dGTP et le c7dATP diffèrent respectivement de dGTP et dATP, par le changement de l'azote en position 7 par un carbone. Ce changement conduit à la disparition de l'accepteur N7 dans le grand sillon, mais l'appariement des paires de bases de type Watson-Crick n'est pas modifié par ce changement, à la différence de l'appariement de type Hoogsteen où N7 est impliqué.

Ces 4 analogues offrent une palette de modification d'appariement, de géométrie d'appariement et d'interaction avec les molécules d'eau et les éléments protéiques. dans le grand et petit sillon

Les sites de terminaison ont été identifiés lors de la polymérisation catalysée par la reverse transcriptase de HIV-1 et du fragment de Klenow en changeant un des dNTPs par son analogue de base. dTTP a été remplacé par dUTP, dGTP par dITP ou c7dGTP et dATP par c7dATP. Dans cette étude, la synthèse est amorcée par pbs.

La réaction débute par formation du complexe réactionnel entre la matrice/amorce marquée à l'extrémité 5' (0,05 pmoles) avec l'enzyme (0,01 U/µl) par incubation à 37°C pendant 3 min. pour les 3 RTs et à 25°C pour le fragment de Klenow. Puis une solution contenant les 3 dNTPs et un des analogues à la concentration finale de 500 µM chacun et l'ADN piège (100:1 : ADN piège : matrice/amorce froid) est ajouté. L'utilisation d'ADN piège permet d'empêcher la réinitialisation de la polymérisation au niveau des sites de terminaison. En parallèle un contrôle en présence des 4 dNTPs "normaux" a été effectué dans des conditions réactionnelles identiques à la réaction en présence d'analogues. L'ensemble est incubé 30 min. à 37°C pour la reverse transcriptase de HIV-1 et à 25°C pour le fragment de Klenow. La réaction est stoppée par ajout de 1/3 de volume de solution stop (v/v), puis les produits de réaction sont déposés sur gel d'acrylamide 13% dénaturant 8 M urée. La position des sites de terminaison est identifiée par la co-migration des produits de séquençage (méthode de Sanger).

Chaque réaction a été effectuée 3 fois, des variations dans l'intensité des taux de terminaison sont observées mais elles sont faibles et inférieures à 10%. Pour la reverse transcriptase de HIV-1 et le fragment de Klenow, les taux de terminaison de la synthèse avec les différents analogues (bâtons noirs) en fonction de la séquence sont reportés en présence des taux de terminaison obtenus lors de la réaction de contrôle c'est-à-dire en présence des bases "normales" (bâtons blancs).

### 2.1. Identification des sites de terminaison avec la reverse transcriptase de HIV-1

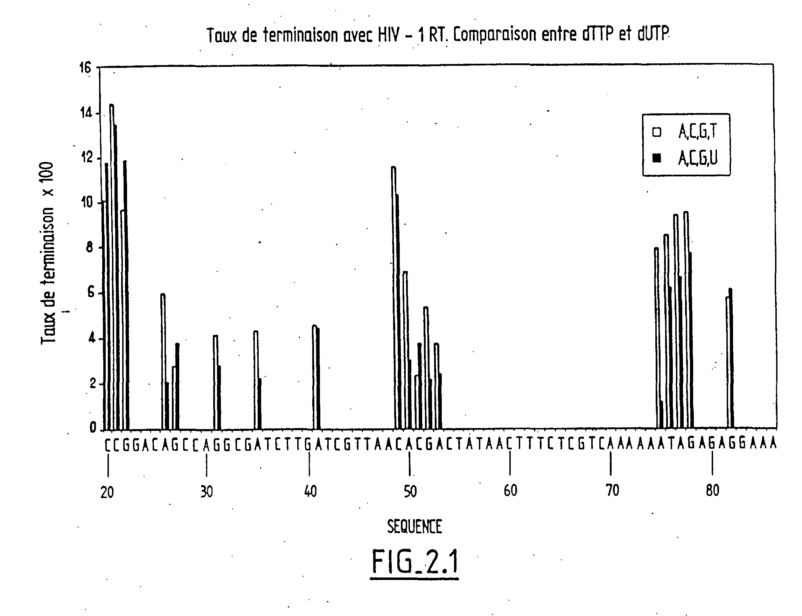

### 2.1.1. Identification des sites de terminaison en présence de dUTP (Figure 2.1)

Très généralement, le taux de terminaison avec dUTP est plus faible qu'avec dTTP. Les sites qui présentent une diminution de plus de la moitié de leur taux de terminaison en présence de dUTP au lieu de dTTP, sont au nombre de 7, dont 6 ont la particularité de succéder à l'incorporation de dUMP ( c'est-à-dire que les sites sont positionnés sur un résidu A sur la matrice : A26, A35, A50, A53, A75 et A77), le septième s'arrête sur un résidu G en position 52 qui précède l'incorporation d'un U. D'autres sites de terminaison situés juste en amont d'un résidu A sur la matrice ont tendance également à voir leur taux d'avortement diminuer, mais de façon moins importante : C49, T76 et G78. Certains sites, non localisés en amont ou sur un résidu A voient leur intensité augmenter : G22 et C52.

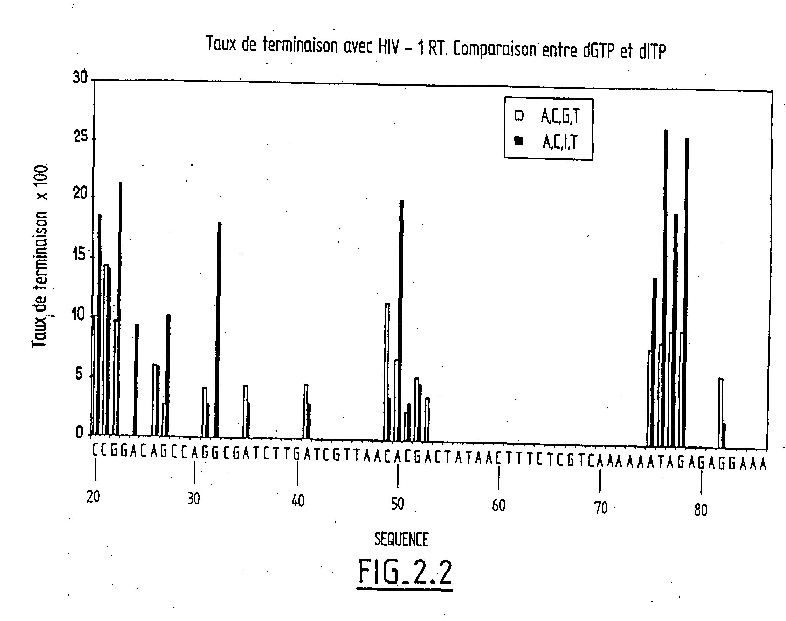

### 2.1.2. Identification des sites de terminaison en présence de dITP (Figure 2.2)

En général, le taux d'arrêt est plus fort avec dITP par rapport à dGTP. Le changement de dGTP par DITP, un de ses analogues, lors de la réaction catalysée par la reverse transcriptase de HIV-1 (Figure 2.2) dans le milieu réactionnel conduit à une augmentation importante (d'un facteur proche de 4) des taux de terminaison de 10 sites. Sur ces 10 sites, 5 précédent l'incorporation du dITP dans le brin polymérisé (C20, A24, G27, G32 et A50), 4 sont situés dans le quadruplet juste à la sortie de l'homo-oligomère (dA)6 et le dernier est situé sur le résidu G22. Mais d'autres sites voient leur intensité diminuer en présence de dITP parmi lesquels les résidus G31, A35, A41, G82, C49 et A53, les trois premiers sites n'ont pas dans leur environnement matriciel proche de résidu C, tandis que C49 est un site d'incorporation du dITP et A53 est localisé en amont d'un résidu C. Deux sites nouveaux apparaissent : devant C25 et C39 de la matrice.

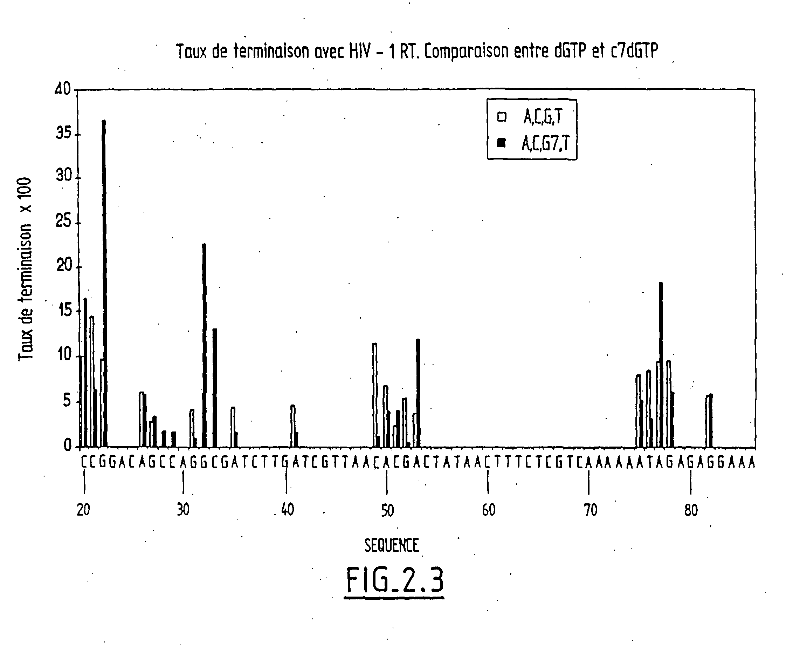

### 2.1.3. Identification des sites de terminaison en présenée de c 7dGTP (Figure 2.3)

Sur les 18 sites de terminaison obtenus en présence des bases "normales", 6 sont augmentés considérablement (jusqu'à 3,5 fois), d'autres sont diminués jusqu'à 10 fois et 4 sites nouveaux apparaissent. Certains sites de terminaison non détectables avec dGTP, sont mis en évidence avec c7dGTP, certains de ces sites pouvant atteindre des pourcentages considérablement élevés : ces sites sont localisés au niveau des bases G32, C33, C28 et C29, le premier site précède l'incorporation de c7dGTP, tandis que les trois derniers sites cités sont en position où c7dGTP est incorporé dans le brin polymérisé. Le taux de terminaison est augmenté en face de la plupart des C sur la matrice sauf C21 et C49 qui présentent respectivement 10 % et 90% de diminution.

D'autres sites voient leur intensité augmenter, mais dans une moindre mesure : en face de A53 (qui précède l'incorporation du c7dGTP) et G22 et A77, ce dernier étant localisé à la sortie de la suite de 6A. D'autres sites voient leur taux de terminaison diminuer alors qu'ils ne sont localisés ni sur un site d'insertion de c7dGMP ou d'extension suivant cette insertion.

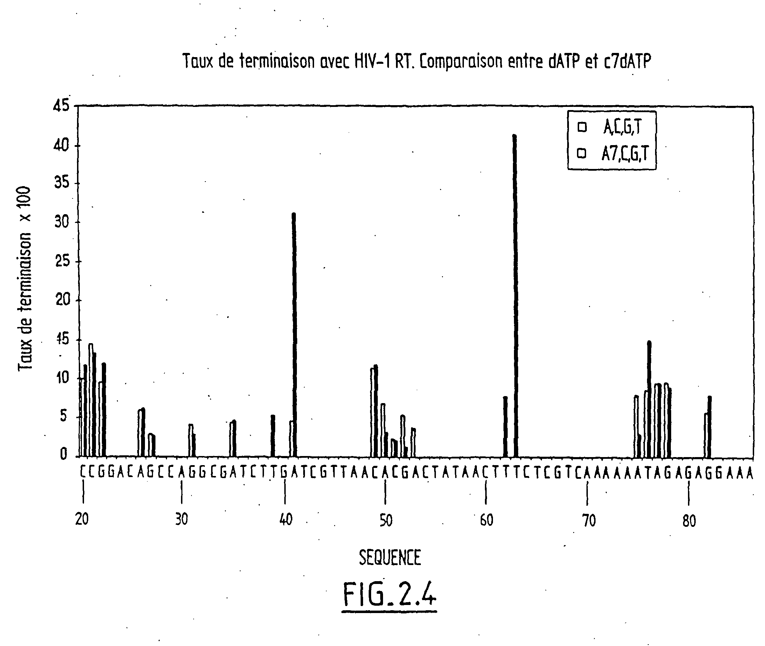

### 2.1.4. Identification des sites de terminaison en présence de c7dATP (Figure 2.4)

Les taux de terminaison en présence de c7dATP au lieu de dATP ont des comportements contrastés : certains des sites, observés avec dATP voient leur intensité augmenter de façon remarquable : en face de A41 (x7) et T76 (x2). D'autres, au contraire, diminuent (face aux bases A50, A53, G52 et A75). Le plus remarquable est l'apparition de sites de terminaison nouveaux face à T39, T62 et surtout T63 qui arrête près de la moitié des complexes d'élongation. Le site fortement augmenté (face à A41) précède l'incorporation de c7dATP. Les 3 sites nouveaux sont des sites d'incorporation de c7dATP consécutifs mais d'autres sites consécutifs ne donnent pas lieu à des arrêts (T45 et T46 par exemple).

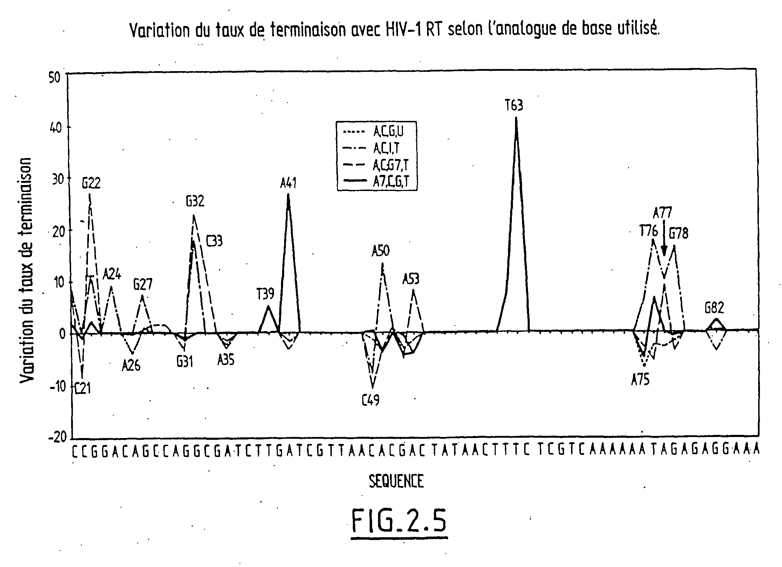

### 2.1.5. Comparaison des sites de terminaison suivant l'analogue utilisé. (Figure 2.5)

Les variations des taux de terminaison avec les différents analogues par rapport aux taux obtenus en présence des bases "normales" sont résumées dans la figure 2.5.

Chaque analogue de base induit des modifications dans les taux de terminaison de la polymérisation catalysée par la reverse transcriptase de HIV-1.
- L'échange de dTTP par dUTP diminue de façon, en général, modestement (maximum 5%) le taux de terminaison de la plupart des sites localisés avant et après l'incorporation du dUTP. Sont principalement concernées les bases en aval de la position 50 et les 6A.
- Le dITP induit une augmentation forte de la terminaison, sauf en 2 sites ( en 49 et 82, où il y a un peu moins d'arrêts). Tous les sites de terminaison sont localisés avant l'incorporation du dIMP, c'est-à-dire situés avant un résidu C sur la matrice à l'exception de A53. L'extension d'une amorce ayant à son extrémité 3' un dIMP est modifiée par rapport à l'extension à partir d'un dGMP, surtout sur C49 qui présente une très forte diminution du taux de terminaison et C20 dont l'intensité augmente en présence de dITP. Les deux autres sites C21 et C51 ne présentent pas de variation significative de leur taux de terminaison. De façon surprenante, des sites de terminaison, ne présentant pas de résidu C dans leur environnement matriciel proche, présentent des variations dans le taux de terminaison, en particulier à la sortie de la suite de 6A où l'intensité est en moyenne doublée.
- L'échange de dGTP et de dATP respectivement par c7dGTP et c7dATP conduit à une augmentation de la terminaison de la polymérisation à partir de brins néosynthétisés ayant à leur extrémité 3' une base modifiée, à l'exception de C21 et de C49 dans le cas de c7dGTP. Les sites localisés avant l'incorporation de bases modifiées, présentent une augmentation du taux de terminaison dans le cas de c7dGTP, à l'exception de A50. La variation des taux de terminaison induite par le changement de dATP par c7dATP est plus complexe : alors qu'au niveau de A41, le taux de terminaison est augmenté de près de 7 fois en présence de c7dATP, sur A75 elle diminue de près de 2 fois et sur A35 elle reste stable. Certains sites non localisés sur ou avant l'incorporation de bases modifiées présentent une différence de taux de terminaison par rapport à la base "naturelle". La plupart de ces sites voient leur taux de terminaison diminuer en présence de c7dNTP au lieu de dNTP, à l'exception de G23 et A77 dans le cas de c7dGTP. Ces positions ont la particularité de précéder l'incorporation d'un résidu C sur la matrice, alors que tous les autres sites qui présentent une diminution de l'intensité de terminaison, précèdent l'incorporation d'une pyrimidine ou d'un T à l'exception de G31. Dans le cas de c7dATP, la première base modifiée à être incorporée dans le brin polymérisé est en position T36, en amont de cette position, la terminaison de la polymérisation catalysée par la reverse transcriptase de HIV-1 n'est pas modifiée qu'il y ait dATP ou c7dATP dans le milieu réactionnel.

Les quatre analogues étudiés ont des effets contrastés sur la polymérisation :

L'échange de dTTP par dUTP ne modifie que très peu les taux d'arrêts, avec un tendance générale à la diminution surtout au niveau de 6A.

L'échange de dGTP par dITP introduit des arrêts nouveaux importants et en général une augmentation conséquente des taux de terminaison en début d'élongation, autour de +50 et surtout après 6A.

L'échange de dGTP par c7dGTP introduit deux sites nouveaux importants et une augmentation importante en début d'élongation, puis une tendance nette à la diminution du taux de terminaison.

L'échange de dATP par c7dATP a globalement peu d'effet, sauf une augmentation importante devant T(matrice)42 et trois nouveaux sites, dont l'un sur la dernière base d'un triplet T(matrice)61-63, où s'arrêtent pratiquement la moitié des complexes d'élongation. La terminaison au niveau des 6A est peu affectée. Il semble que c'est l'accumulation locale de T qui conduit à augmenter la terminaison.

Bien que fréquents, les arrêts devant les sites où la base modifiée est demandée ne sont pas systématiques, que cette demande intervienne de façon isolée ou non.

Globalement les analogues (sauf dUTP) augmentent les taux de terminaison dès l'initiation de l'élongation.

### 2.2. Identification des sites de terminaison avec le fragment de Klenow

Globalement la présence des analogues de base réduit l'efficacité de la polymérisation du fragment de Klenow, rendant la détection des sites de terminaison difficilement décelable au-delà de la position +55, c'est la raison pour laquelle nous n'avons représenté dans les différentes figures qui suivent que les sites de terminaison localisés entre les position +20 et +55.

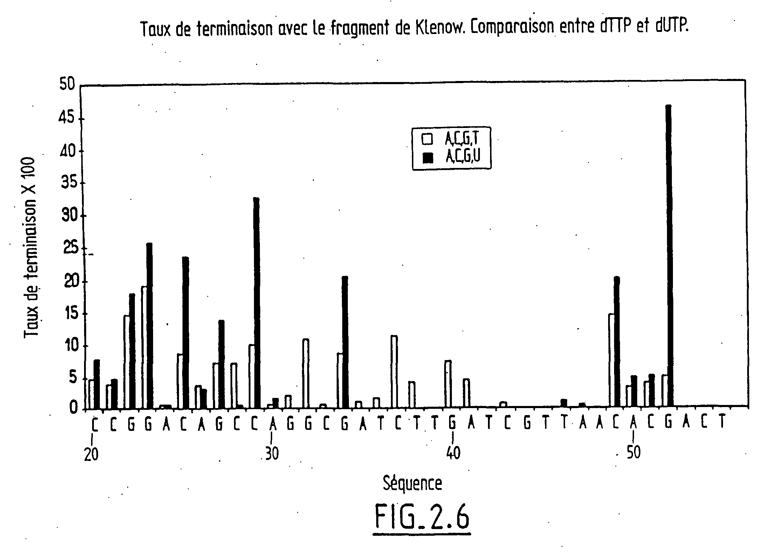

### 2.2.1. Identification des sites de terminaison en présence de dUTP (Figure 2.6)

L'échange de dTTP par dUTP augmente de façon significative le taux de terminaison de la plupart des sites qui précédent l'incorporation du dUMP au sein du brin néosynthétisé, à l'exception de G40. A part G34, les sites de terminaison en présence de dUTP ne sont plus décelables entre 31 et 45.

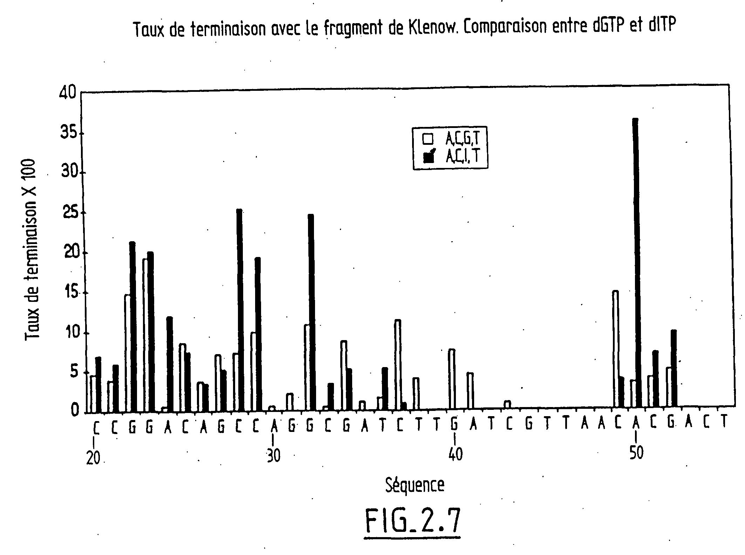

### 2.2.2. Identification des sites de terminaison en présence de dITP (Figure 2.7)

Les augmentations des taux de terminaison des sites localisés avant l'incorporation d'un dITP (A24, C28, G32, T36 et A50) sont en général plus fortes que ceux où le dITP vient d'être incorporé (29, C33, C51). Il y a moins d'arrêt au niveau de C25, C37 et C49 : ces sites ont la particularité de précéder l'incorporation de A ou T au sein du brin polymérisé, alors que tous les autres sites précédent l'incorporation d'un G ou d'un C, à l'exception de C29 qui a la particularité d'être précédé de l'incorporation de 2 dIMP.

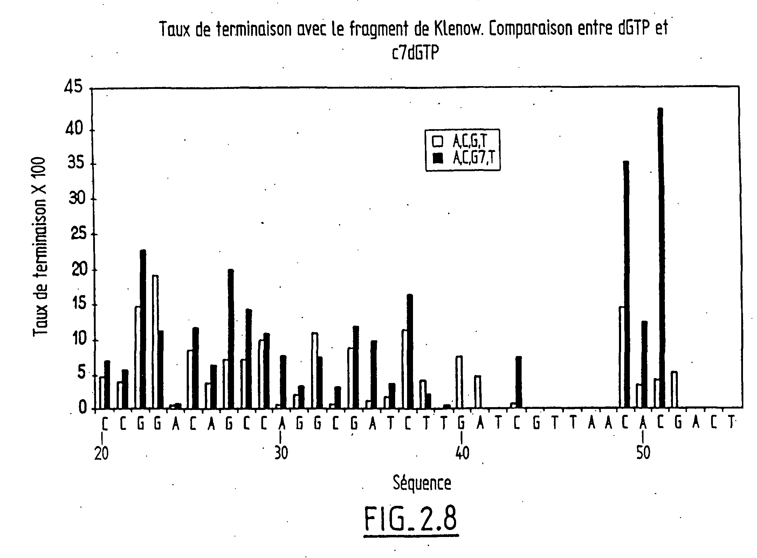

### 2.2.3. Identification des sites de terminaison en présence de c 7dGTP (Figure 2.8)

Tous les arrêts sur les sites où le c7dGMP est incorporé dans le brin polymérisé sont en augmentation par rapport à la réaction avec le dGTP, ainsi que (mais dans une moindre mesure) les sites précédant l'incorporation du c7dGMP, à l'exception de C32 dont le taux de terminaison diminue de 26% en présence de c7dGTP. D'autres sites non localisés en amont ou sur des sites d'insertion du c7dGMP présentent également des variations du taux de terminaison.

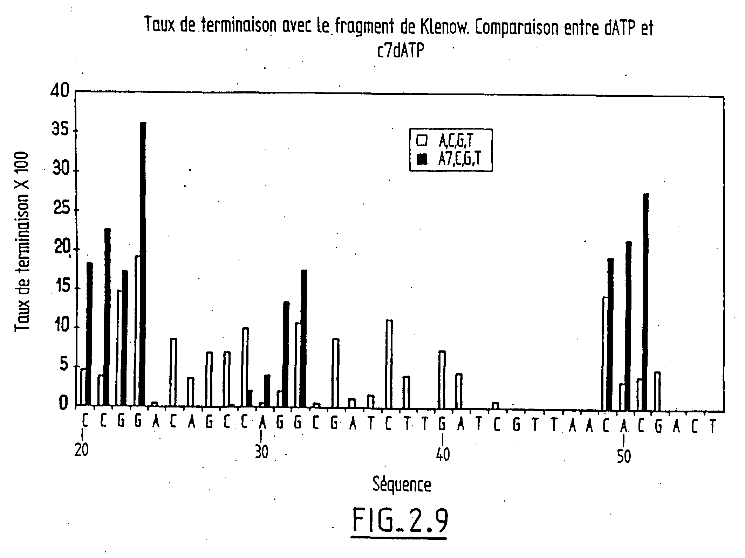

### 2.2.4. Identification des sites de terminaison en présence de c7dATP (Figure 2.9)

Il y a augmentation des taux de terminaison en 3 vagues, au début de l'élongation, après 1 tour d'hélice et 3 tours d'hélice. Il n'y a aucune corrélation avec la présence de T sur le brin matrice. Les sites de terminaison compris entre les 3 vagues observées avec le dATP, ne donnent pas lieu à des arrêts avec l'analogue c7dATP.

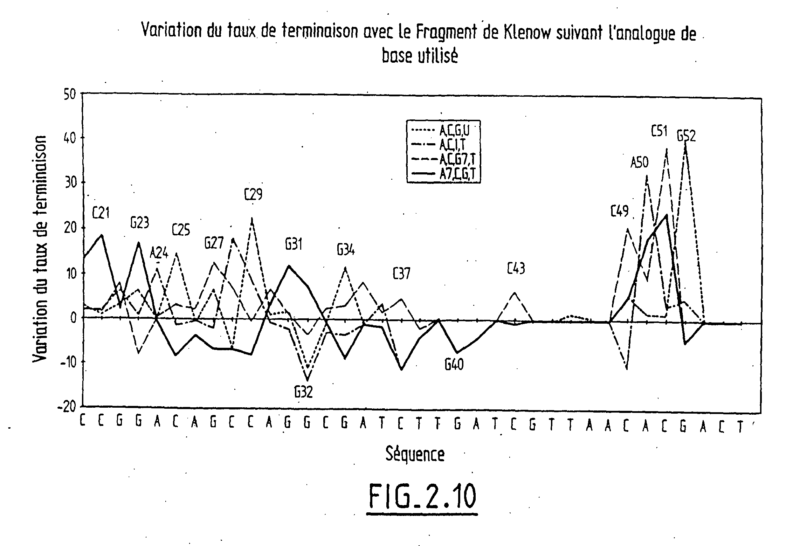

### 2.2.5. Comparaison du taux de terminaison suivant l'analogue (Figure 2.10)

Comme nous pouvons le voir sur la figure 2.10, les effets des 4 analogues sont importants en début d'élongation et jusqu'à environ +40, les arrêts augmentent ou diminuent tour à tour. La région +50 est le siège de très importantes augmentations avec les 4 analogues.

### Exemple 3 : Comparaison de l'influence de la présence des analogues de bases sur la terminaison de la synthèse catalysée par les deux enzymes

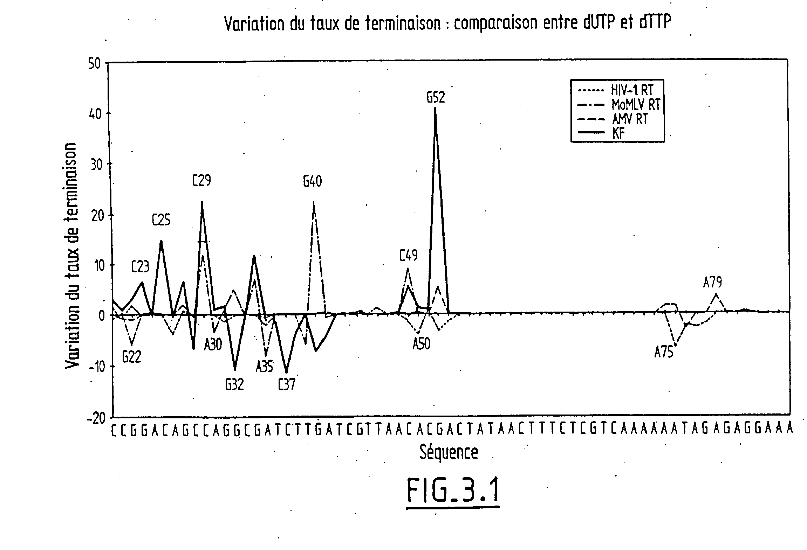

### 3.1. Récapitulation de l'influence du dUTP sur la terminaison avec le fragment de klenow de l'ADN polymérase et la reverse transcriptase de HIV-1 (Figure 3.1)

La différence structurale entre dUTP et dTTP est l'absence de groupement méthyle en position C5 au niveau de la base pyrimidique. Ce groupement n'est pas impliqué dans l'appariement des bases, mais il modifie le grand sillon de la double-hélice de l'ADN, de plus la méthylation des bases conduit à une augmentation modérée de l'interaction entre les bases.

La reverse transcriptase de HIV-1 et le fragment de Klenow ont un comportement différent vis-à-vis du dUTP. Sur les résidus A de la matrice à copier, correspondant aux sites où U a été incorporé, les taux de terminaison diminuent pour la reverse transcriptase. Dans la séquence étudiée et les conditions de l'expérience, l'extension de l'amorce ayant à son extrémité 3' un U au lieu de T est donc en général facilitée avec la reverse transcriptase de HIV-1, bien que globalement la variation est modeste. Lors de la synthèse catalysée par le fragment de Klenow, la variation de l'intensité de la terminaison est plus faible au niveau de tels sites.

L'effet de la base matrice en 3' de A est en général d'augmenter le taux de terminaison avec dUTP avec le fragment de Klenow, alors que dans le cas de la reverse transcriptase de HIV-1, elle diminue. L'incorporation sur la base suivant celle du U est en général moins susceptible de terminaison (reverse transcriptase de HIV-1) et pratiquement sans effet pour le fragment de Klenow.

Certaines terminaisons non contiguëes à l'incorporation de U sont également perturbées par la présence du dUTP au lieu du dTTP. Ainsi les sites localisés deux bases en amont du U incorporé présentent en général une variation des taux de terminaison avec les 2 enzymes, ce phénomène est plus perceptible dans le cas de la reverse transcriptase de HIV-1.

Certains sites de terminaison non localisés sur ou en amont d'un site d'insertion du dUMP lors de la polymérisation catalysée par le fragment de Klenow présentent également des variations dans leur taux de terminaison, la majorité de ces sites présentent une diminution du taux de terminaison en présence de dUTP, à l'exception des 3 premières bases polymérisées alors qu'aucun dUMP n'a été incorporé dans le brin polymérisé et de G27.

L'effet de l'incorporation de U à la place de T est perceptible surtout dans l'élongation correspondant aux deux premiers tours d'hélice (matrice 20 à 40). Les complexes avec la reverse transcriptase de HIV-1 terminent moins fréquemment. Les terminaisons avec KF alternent entre augmentation et diminution. Les mêmes observations valent pour le début du 4^{ème} tour d'hélice, alors que la synthèse du 3^{ème} tour d'hélice se fait sans modification.

Il apparait que l'absence de CH₃ dans le grand sillon facilite légèrement l'élongation de la reverse transcriptase de HIV-1.

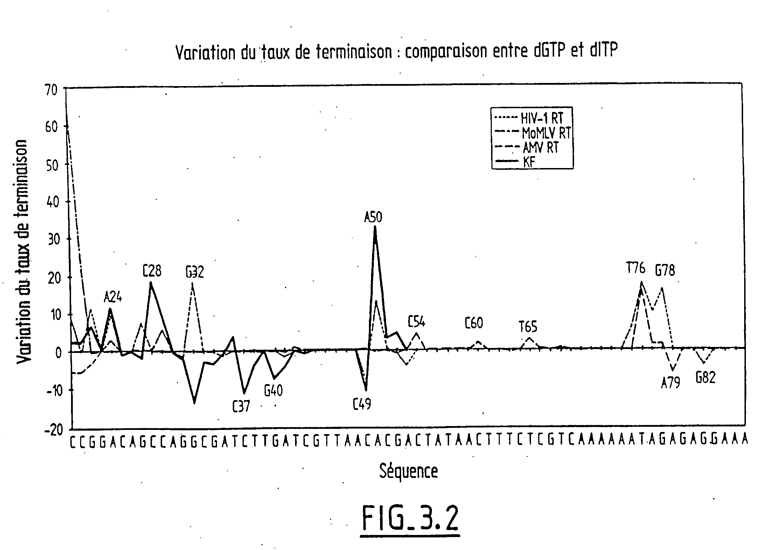

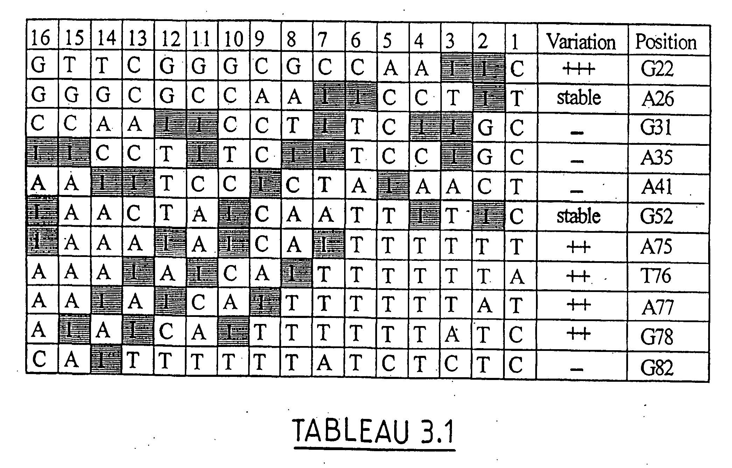

### 3.2. Récapitulation de l'influence du dITP sur la terminaison avec la reverse transcriptase de HIV-1 et du frirgment de Klenow (Figure 3.2)

La différence structurale entre dITP et dGTP est l'absence du groupement NH2 impliqué dans l'appariement avec la base C, conduisant, entre autres, à une diminution de l'interaction entre les bases I:C par rapport à G:C.

Les sites de terminaison obtenus lors de la synthèse catalysée par la Reverse transcriptase de HIV-1 et du fragment de Klenow, précédant l'incorporation de I présentent une augmentation de leur intensité, à l'exception de A53 pour la reverse transcriptase et de G27 pour la fragment de Klenow. L'augmentation du taux de terminaison est également de règle sur les sites C avec le fragment de Klenow (à l'exception de C25, C37 et C49), tandis qu'avec la reverse transcriptase de HIV-1, l'influence de la présence du dITP est très contrastée : certains sites ne présentent pas de différence d'intensité significative C21 et C51, alors que l'amplitude de C49 est fortement réduite et celle de C20 augmente de façon dramatique pour la polymérisation, nous ne pouvons exclure dans le cas de C20 que la difficulté à incorporer le dITP au niveau de C21 ne soit responsable de cette augmentation.

Tous les sites C sur la matrice qui présentent une diminution du taux de terminaison avec le fragment de Klenow et la reverse transcriptase de HIV-1 précédent l'incorporation d'un A ou d'un T, alors que les sites C dont le taux de terminaison augmente en présence de dITP au lieu du dGTP ont la particularité de précéder l'incorporation d'un C ou d'un G.

La moindre stabilité de la paire I.C, l'absence pour cette paire d'accepteur de proton dans le petit sillon ont un effet inhibiteur marqué sur l'élongation par la reverse transcriptase de HIV-1 durant la synthèse du premier tour d'hélice. A la sortie des 6A, la reverse transcriptase de HIV-1 subit une inhibition forte.

Un fait surprenant observé dans le cas de la reverse transcriptase de HIV-1 est la différence d'intensité observée sur certains sites qui n'ont pas dans leur environnement proche de résidu C et qui pourtant présentent dans certains cas une variation considérable de la terminaison. La séquence du brin néosynthétisé de ces sites de terminaison jusqu'à la 16^{ème} base a été représentée dans le tableau 3.1 pour la reverse transcriptase de HIV-1.

Pour les sites de terminaison non localisés avant ou sur un site d'insertion du dITP, le taux de terminaison diminue lors de la polymérisation avec la reverse transcriptase de HIV-1, à l'exception de G22 qui présente une très forte augmentation de l'intensité de terminaison, et A26 et A41 qui restent stables. L'extraordinaire augmentation du taux d'avortement au niveau de G22 pourrait s'expliquer par la variation de la conformation de la double-hélice entre les position +2 et +5 du brin amorce/ matrice qui par le changement de dGTP par dITP conduit à une suite de A-I au niveau de la forme A de matrice/amorce.

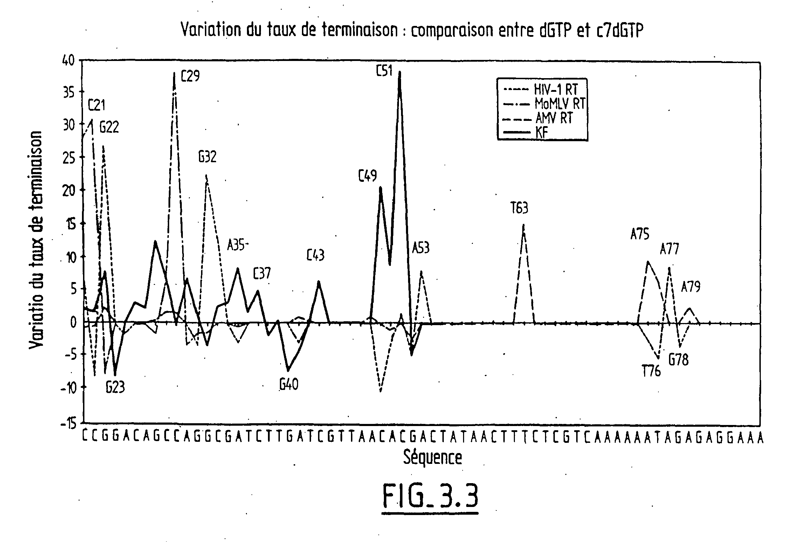

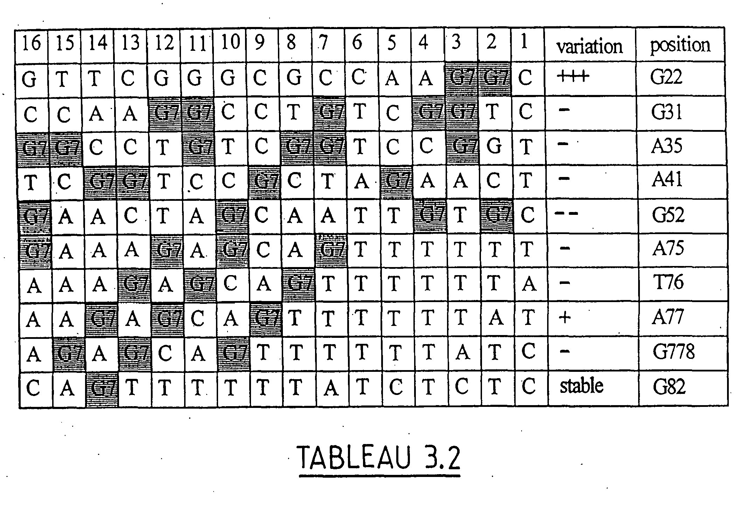

### 3.3. Récapitulatif de l'influence du c7dGTP sur la terminaison avec le fragment dé klenow et la reverse transcriptase de HIV-1 (Figure 3.3)

La substitution de dGTP par c7dGTP ne devrait pas modifier substantiellement l'appariement c7G:C et a pour effet principal de supprimer un accepteur de proton dans le grand sillon donc l'interaction de l'ADN avec l'enzyme, mais elle provoque des inhibitions d'élongation très importantes dès la synthèse du 1^{er} tour d'hélice pour la reverse transcriptase de HIV-1.

Avec la reverse transcriptase de HIV-1, et le fragment de Klenow, les sites qui voient leur taux de terminaison augmenter sont dans leur grande majorité des sites localisés sur des sites d'incorporation du c7dGTP ( à l'exception de C21 et C49 avec la reverse transcriptase de HIV-1, les deux premières bases polymérisée), ainsi que les positions qui précédent l'insertion du c7dGMP au sein du brin polymérisé, à l'exception de A50 avec la reverse transcriptase de HIV-1 et G32 avec le fragment de Klenow.

D'autres sites non impliqués dans l'incorporation ou l'extension du c7dGMP présentent également des variations dans leur taux de terminaison, en particulier au niveau des sites localisés à la sortie de la suite de 6A qui présentent globalement une diminution de la terminaison dans le cas de la reverse transcriptase de HIV-1.

Les sites qui présentent des variations de l'intensité des sites de terminaison en présence du c7dGTP avec la reverse transcriptase de HIV-1 ont été répertoriés dans le tableau 3.2. Dans le cas de la reverse transcriptase de HIV-1, avec le c7dGTP seuls deux sites présentent une augmentation du taux de terminaison G22 et A77, un seul reste stable : G82 et les autres présentent une diminution. Au niveau de la position 22, seuls les résidus en +2 et +3 sont des c7dGMP.

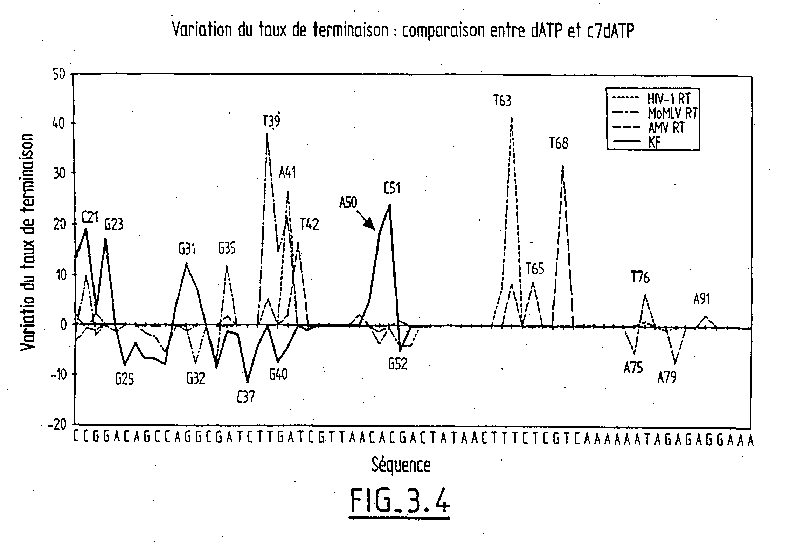

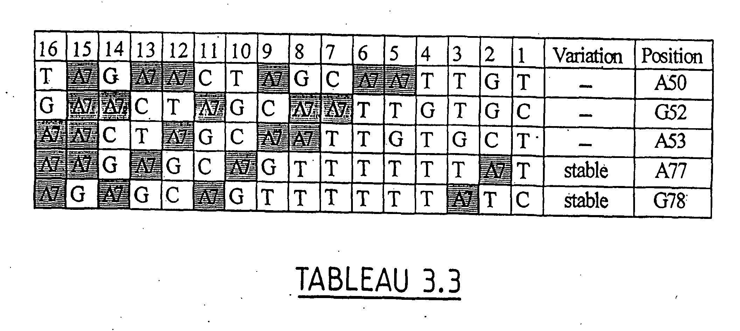

### 3.4. Récapitulatif de l'influence du c7dATP sur la terminaison de la reverse transcriptase de HIV-1 et du fragment de klenow (Figure 3.4)

Comme le précédant analogue étudié, cette substitution ne devrait pas affecter l'appariement de c7A.T, cependant elle perturbe considérablement l'élongation par la RT, surtout au cours de la synthèse du 2^{ème} tour d'hélice, au début du 5^{ème} et pendant le 6^{ème}, alors que la synthèse du 1^{er} tour est relativement peu affectée.

Dans le cas de la reverse transcriptase de HIV-1, les sites qui voient leur taux de terminaison augmenter sont, dans leur grande majorité des sites localisés sur ou en amont des résidus T sur la matrice, impliquant que la polymérase a des difficultés à incorporer le c7dATP au sein du brin polymérisé ; une fois le c7dATP incorporé au sein de la matrice, l'extension est également réduite à l'exception de A75.

Le cas du fragment de Klenow est particulier dans la mesure où des variations de l'intensité de la terminaison sont observées lors de la polymérisation des premières bases alors qu'aucun c7dAMP n'a été incorporé. Ces variations sont également présentes en présence de seulement 3 des 4 dNTPs (C,G,T) (données non présentées).

Comme dans les cas avec dITP et c7dGTP nous avons répertorié pour la reverse transcriptase de HIV, dans le tableau 3.3, les sites présentant des différences dans leur taux de terminaison avec dATP et c7dATP, alors qu'ils ne sont ni localisés ni sur une position d'incorporation ni d'extension de l'analogue.

Avec la reverse transcriptase de HIV-1, des variations de l'intensité de la terminaison sont observées sur des sites non impliquées dans l'extension ou dans l'incorporation de c7dATP, ces variations sont probablement dues à la différence de la séquence du brin néosynthétisé possédant des c7dAMPs au lieu des dAMPs (voir tableau 3.3) qui modifieraient l'interaction des RTs avec le complexe réactionnel et donc soit augmenterait ou diminuerait le fréquence de dissociation.

### 3.5. Récapitulatif de l'influence des analogues de bases sur la terminaison

Chaque analogue de base induit des modifications dans la polymérisation catalysée par la reverse transcriptase de HIV-1 et le fragment de klenow. Un fait intéressant dans ce chapitre est l'influence de ces analogues sur des sites non impliqués dans l'incorporation ou l'extension de base modifiée, à l'exception de U. Or tous ces sites qui présentent une variation dans leur taux de terminaison, ont la particularité d'être précédés de l'incorporation de bases modifiées sur le brin polymérisé, à l'exception du fragment de klenow avec c7dATP. Les variations des taux de terminaison au niveau des premières bases polymérisées en présence de c7dATP pourraient s'expliquer par l'activité exonucléasique 3'→5' du KF.

### Exemple 4 : Etude de la fidélité de la reverse transcriptase de HIV-1 en présence des analogues de base

Pour comprendre le rôle des trois dNTP présents impliqués dans la misincorporation suite à l'absence du quatrième dNTP, le dGTP ou/et le dCTP ont été respectivement remplacés par le dITP et le dUTP dans la réaction moins A. Le dITP a été choisi car I peut s'apparier avec T et donc suppléer l'absence de A, et dUTP a été choisi pour évaluer l'effet que l'absence du méthyle d'un T peut avoir sur la fidélité d'incorporation de la paire voisine. Pour cette raison nous nous intéressons non seulement aux sites et taux de misincorporations, mais à la cinétique avec laquelle ces misincorporations se font. Les conditions les plus propres à l'absence de fidélité de la HIV-RT ont été retenues : utilisation de la matrice + env (amorce ppt) et absence de dATP (données non montrées).

Après hybridation de l'amorce ppt marquée à l'extrémité 5' (1 pmole) avec la matrice + env (2 pmoles), la reverse transcriptase de HIV-1 à la concentration finale de 0,1U/µl est ajoutée. Des fractions équivalentes (1/4 du mélange) sont additionnées de solutions contenant dCTP, dGTP, dCTP (témoin), dCTP, dITP, dTTP ou dCTP, dGTP, dUTP ou dCTP, dITP, dUTP à la concentration finale de 500 µM chacun. Des aliquotes de 1/4 du volume réactionnel sont prélevés au bout de 1min, 2,5 min, 5 min et 10 min et la réaction est stoppée par 1 mM EDTA (concentration finale). Après dénaturation à 92°C pendant 5 min, les produits des réactions sont déposés sur gel d'acrylamide 13% dénaturant 8M urée.

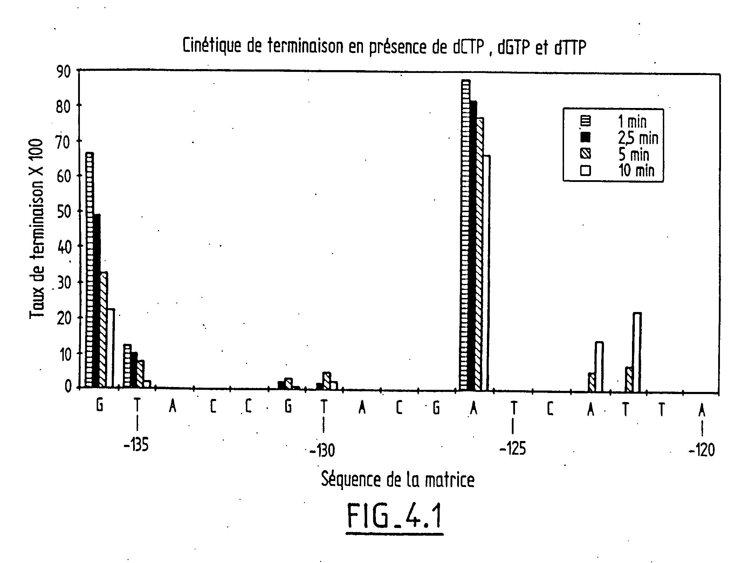

Dans les conditions de l'expérience (concentration d'enzyme 0,1U/µl), en l'absence de dATP mais en présence des 3 autres dNTPs normaux, les pauses majoritaires sont localisées en amont des résidus T sur la matrice (figure 4.1), mais tous ces sites n'ont pas des capacités d'arrêt identiques. Au niveau de la deuxième misincorporation, l'enzyme pause très peu aussi bien en amont de T-130 (matrice) que sur T-130, alors qu'au niveau de T-135 et T-125 (matrice), l'enzyme pause majoritairement en amont des bases et peu sur l'extrémité mal appariée.

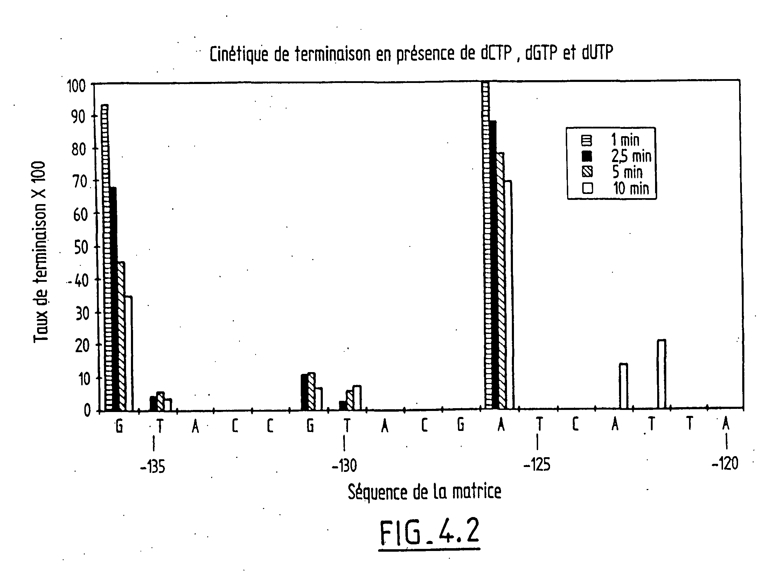

En remplaçant le dTTP par le dUTP, le profil des pauses est peu changé (figure 4.2), bien qu'une augmentation du taux de terminaison soit visible par rapport. à la réaction avec le dTTP.

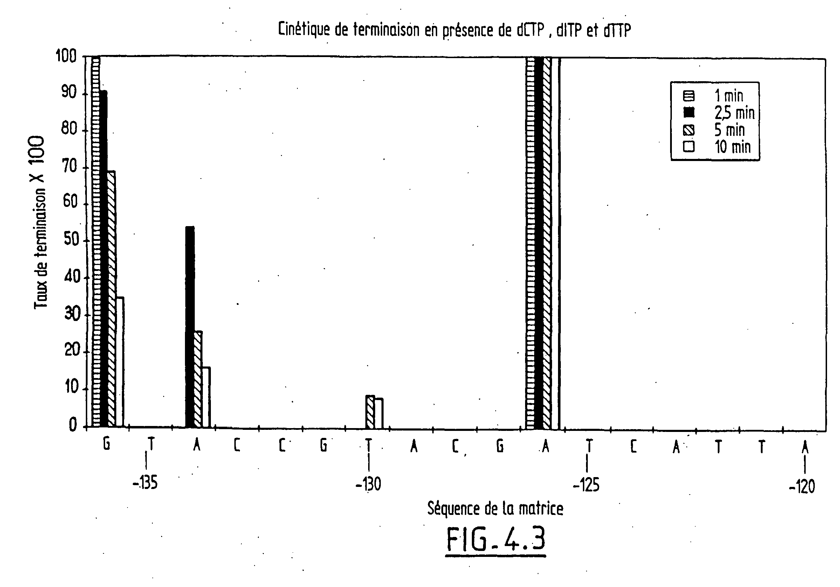

En remplaçant le dGTP par le dITP, la pause devant le T-135 (matrice) n'est plus détectable , alors qu'une pause apparait face au A -134 (figure 4.3), cela bien que le dTTP soit présent. La terminaison devant T-130 (matrice) diminue fortement d'intensité.

Le changement simultané de dTTP et de dGTP par dUTP et dITP ne modifie pas de façon significative les profils des pauses par rapport à la réaction avec dITP, mais augmente l'intensité des pauses.

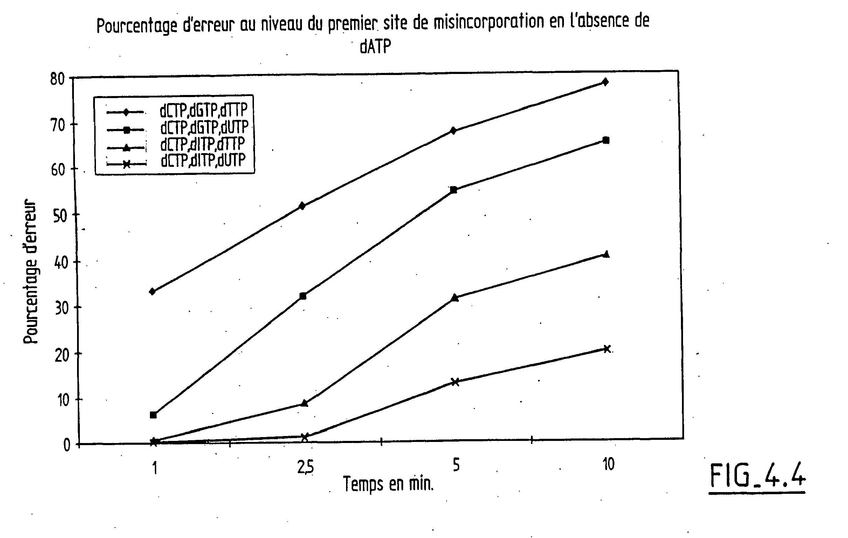

La figure 4.4 représente la variation du taux de misincorporation devant T-135 (matrice), le premier site de misincorporation, en fonction du temps et de l'analogue de base utilisé.

En présence de dCTP, dGTP et dTTP, le taux d'erreur au niveau de la première misincorporation face à T-135 augmente de 33% à 78% au bout de 10 min d'incubation, ce pourcentage reste ensuite constant (80% d'erreur au bout de 30 min). L'échange de dTTP par dUTP conduit à une translation de la courbe, le taux d'erreur grimpe de 5% à 65% après 10 min d'incubation (après 45 min, ce pourcentage tend vers 77%, expériences non présentées).

Le remplacement de dGTP par dITP, ou de dGTP et dTTP par dITP et dUTP, induisent une augmentation respective des taux d'erreur de 0 à 40 et de 0 à 20% , après 10 min d'élongation ; des valeurs proches sont également observées au bout de 45 min. (données non présentées). Il semble donc y avoir deux effets: un effet cinétique, lié à l'incorporation ralentie de l'analogue dITP, et un moindre taux d'erreur en présence de cette base. Ceci s'explique par la substitution du dITP au dATP absent.

### Exemple 5 : Analyse des misappariements

Pour mieux comprendre les mécanismes qui régissent l'incorporation de nucléotides incorrects insérés lors des réactions "moins ", plusieurs "mutants" obtenus avec la reverse transcriptase de HIV-1 ont été séquencés. L'étude a été limitée au cas de "moins A", car lés séquences mutées sont facilement identifiables par restriction.

### 5.1. En présence des bases "naturelles."

Les différents mutants sont générés au cours de la réaction "moins A" avec la matrice + env, et l'amorce ppt. Matrice/amorce marquée (0,5 pmole), reverse transcriptase (0,04 U/µl) et les 3 dNTPs (500 µM final) ont été incubés 30 mn à 37°C, puis le dATP (500 µM final) a été ajouté et l'incubation poursuivie 30 mn à 37°C. La réaction est alors stoppée par ajout d'EDTA à la concentration de 10mM finale. Les produits de réaction sont récupérés après précipitation alcoolique, digérés par l'enzyme Sac I. Après dénaturation à 90°C pendant 3 mn, le produit est déposé sur gel d'acrylamide 10% dénaturant. Après migration et mise en autoradiographie, les bandes de 200 bases sont récupérées sur le gel, puis purifiés. Ces fragments sont amplifiés par PCR (15 cycles), coupés par Mlu I et Bam H I et insérés dans pBluescript SK + ppt par ligation avec la T4 ADN ligase puis transformation avec XL 1 Blue.

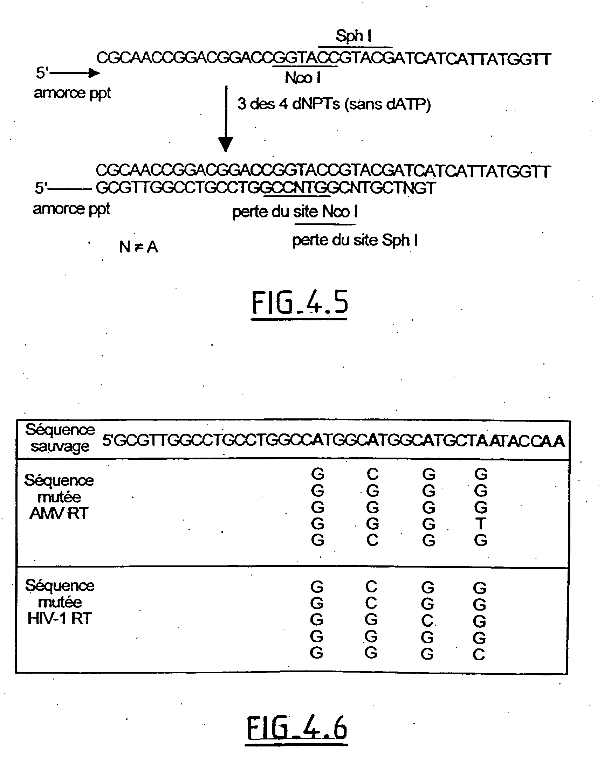

Les plasmides mutés sont facilement sélectionnés car ils n'ont plus le site de restriction reconnu par l'enzyme Nco I (voir figure 4.5). De plus l'absence de digestion enzymatique avec Sph I implique une double mutation.

Avec la reverse transcriptase de HIV-1, sur les 48 plasmides isolés et analysés sur gel d'agarose avec les enzymes de restriction Nco I et Sph I, 33 plasmides ont été mutés à au moins deux sites puisqu'aucun n'est digéré ni par Nco I, ni par Sph I. Aucun plasmide ne possédant qu'une mutation n'a été isolé.

Pour déterminer les types de mutation induites par l'absence de dATP dans le milieu réactionnel lors de la polymérisation catalysée par la reverse transcriptase de HIV-1, certains plasmides ont été séquencés. Les séquences mutées sont représentées dans la figure 4.6. Nous n'avons représenté qu'une partie de la séquence étudiée, le reste de la séquence ne présente pas de mutation.

On constate que dans les conditions de notre étude, et pour les séquences analysées, les erreurs forcées dues à l'absence de dATP dans le milieu ne donne lieu ni à délétion, ni à insertion, mais uniquement à des substitutions de base.

Lors de la première misincorporation G est incorporé de façon exclusive à la place de A sur les 10 plasmides mutés avec la reverse transcriptase de HIV-1, alors que pour les autres positions, bien que le G remplace A préférentiellement, des pyrimidines sont également présentes, en particulier C.

On remarque que la première et deuxième misincorporation s'opère dans un contexte matriciel proche identique (3' GTAC5') mais peut générer des substitutions différentes. Cela implique que le type d'erreur engendrée par la reverse transcriptase de HIV-1 serait affecté par des différences de séquence distante du site de mutation L'erreur engendrée T.dGTP ne peut être expliquée par un glissement de l'amorce ou de la matrice ; aussi cette erreur est probablement engendrée par l'incorporation directe du nucléotide incorrect. En revanche, l'erreur T.dCTP sur le second site pourrait s'expliquer par un tel glissement sur plus d'une base.

### 5.2. Etude en présence d'analogues de bases

La réaction de polymérisation a été effectuée en l'absence de dATP, comme pour la réaction en présence de bases "naturelles", mais le dGTP a été remplacé par le dITP ou le dTTP par le dUTP. Après 30 min à 37°C en présence de la reverse transcriptase de HIV-1 1 et de 3 des 4 bases, la matrice/amorce marquée est purifiée sur colonne Sephadex G50, de manière à éliminer les nucléotides qui n'ont pas été incorporés et en particulier le dITP et le dUTP qui modifierait le site de restriction Sac I. Puis l'élongation est poursuivie pendant 30 min à 37°C en présence des 4 dNTPs naturels à la concentration finale de 500 µM et de l'enzyme (0,05U/µl). Après précipitation alcoolique et digestion avec l'enzyme de restriction Sac I et récupération de la bande de 200 bases, une PCR est effectuée dans les mêmes conditions que pour la réaction en présence des bases "naturelles".

### 5.2.1. Etude de misincorporation par la reverse transcriptase de HIV-1 en présence de dITP

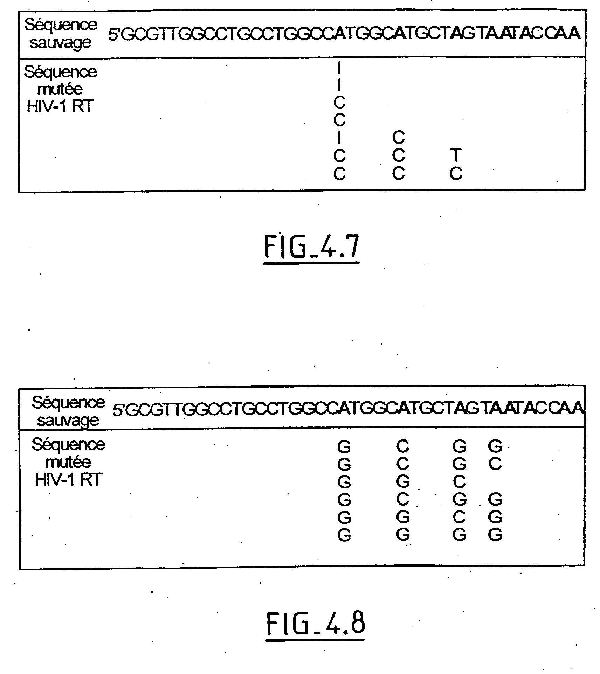

Sur les 36 plasmides isolés, 4 sont mutés sur le premier site où A est demandé au moins, un sur les deux premiers sites et 3 sur les trois premiers sites au moins, d'après le test de digestion enzymatique avec Nco I et Sph I. Les séquences mutées sont représentées dans la figure 4.7.

Aucune délétion, ni insertion n'est détectée par le test enzymatique au sein des 7 plasmides séquencés.

Au niveau du 1^{er} site de misincorporation (T-135 matrice), la base I, analogue de G est incorporé 3 fois, C 4 fois. Au second site (T-130 matrice), C est la seule base trouvée, et au troisième (T-125 matrice) T et C sont détectés.

### 5.2.2. Etude de misincoporation par la reverse transcriptase de HIV-1 en présence de dUTP

Le protocole expérimental est identique sauf que le dTTP a été remplacé par le dUTP.

Sur les 36 plasmides mutés, 21 plasmides présentent au moins deux mutations (déterminer par la digestion enzymatique avec Sph I et Nco I), les 15 autres plasmides sont linéarisés à la fois par les enzymes Sph I et Nco I et ne présentent pas de mutation sur ces sites. La figure 4.8 donne la séquence des 6 plasmides séquencés.

Sur les 6 plasmides séquencés, un seul présente 3 mutations alors que pour les 5 autres, la reverse transcriptase de HIV-1 a incorporé jusqu'à 4 nucléotides incorrects sur le même brin. Tous les mésappariements sur le premier site sont des G, ceux sur les sites suivants sont majoritairement des G aussi, avec des C mais jamais de U. Les mésappariements sont de type T:G mais sont également présents des mésappariements de type T:C mais avec une fréquence d'apparition plus faible (17 contre 6 en considérant tous les mésappariements quelque soit leur position). Mais tous les sites où ont lieu l'incorporation de nucléotides incorrects ne sont pas égaux vis-à-vis du type de mésappariements engendrés par la reverse transcriptase de HIV-1 : au niveau de la 1^{ère} misincorporation, sur lets 6 plasmides mutés, T:G est l'unique mèsàppariement observé, alors qu'au niveau de la 2^{ème} misincorporation T:C est aussi fréquent que T:G. Sur le 3^{ème} et 4^{ème} site de misincorporation, G est introduit préférentiellement par rapport à la base C avec respectivement 4 T:G contre 2 T:C et 4 T:G contre 1 T:C. Aucune base U n'est incorporée pour former le mésappariement T:U.

### 5.2.3. Conclusion

Lors de l'étude des mutants obtenus lors de la réaction moins A en présence des substrats naturels de la reverse transcriptase de HIV-1, les erreurs engendrées sont des substitutions de type transition, la purine A étant préférentiellement substituée par la purine G quand le dGTP est présent dans le milieu réactionnel.

Rappelons que G peut s'apparier à T en appariement "Wobble".

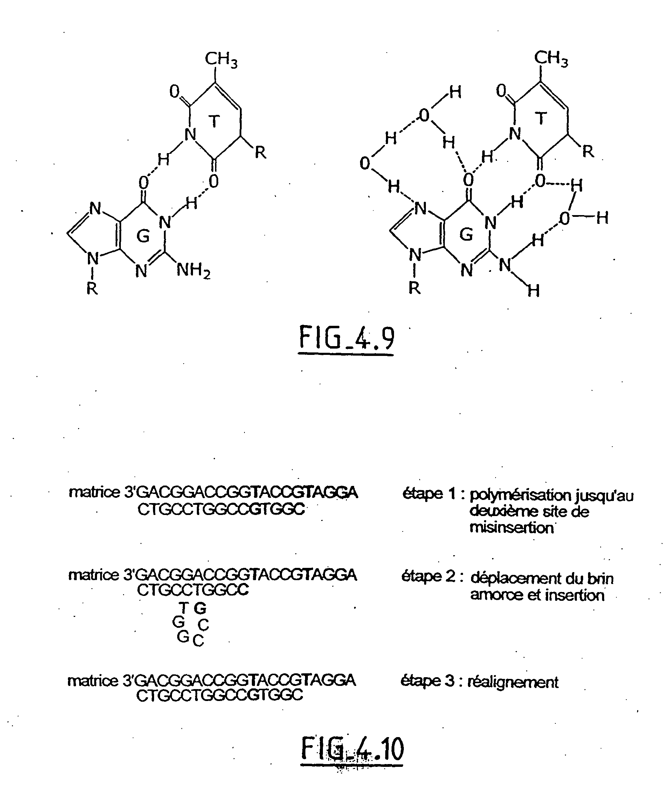

L'appariement G.T, représentée figure 4.9, ne possède pas une géométrie de type Watson-Crick (Hunter et Kennard, 1987). La paire de base G.T n'induit que très peu de perturbations locales de l'hélice et la conformation globale du duplexe est peu affectée. La paire GT donne lieu à des structures d'hydratation complexes (voir figure 4.9) qui permettent de stabiliser l'appariement et l'insertion de cette paire dans la double hélice. La prépondérance de la substitution G peut donc s'expliquer facilement par la stabilité de la paire de base GT.

La paire hétéropyrimidine T:C a été décrite dans deux configurations symétriques seulement, la paire T:T dans la configuration symétrique et assymétrique (Donohue et Trueblood, 1960). C'est seulement dans cette dernière configuration que les positions des liens glycosyles de la paire T:T sont compatibles avec son insertion dans la double hélice. La même observation vaut pour l'une des paires T:C. Les énergies d'interaction entre ces bases sont dans l'ordre T:G>>T:C>T:T (Kudritskaya et Danilov, 1976), soit l'ordre de préférence d'incorporation en face de T (matrice) que nous observons.

Au niveau du premier site de mutation, la substitution par G est systématique. Elle est favorisée sur les autres sites. Les deux premiers sites de mutation possèdent la même séquence locale. Une possibilité pour expliquer la présence du C au second site, mais pas au premier pourrait être trouvée dans le glissement du brin néosynthétisé, facilité par la misincorporation au niveau du premier site (fig 4.10). Mais au niveau des trois autres sites de mutation, la substitution par G ne peut être expliquée par un tel glissement.

En présence de dGTP, dCTP et dTTP, l'ordre de préférence de substitution aux quatre sites est G»C»T lors de la polymérisation catalysée par la reverse transcriptase de HIV-1 en l'absence de dATP.

L'analyse des plasmides mutés se limite aux quatre premiers T (matrice) rencontrés par les enzymes. Dans le cas de la reverse transcriptase de HIV-1, on observe sur le gel d'autoradiographie des extensions bien au-delà. Pour vérifier si les élongations arrêtées sont définitivement terminées ou pausent en restant actives, nous avons refait l'expérience moins A, mais au lieu de stopper la réaction après 30 min à 37°C, le dATP manquant à la concentration finale de 500 µM est additionné au mélange réactionnel et la réaction est stoppée 30 min. plus tard avec 1/3 (v/v) de solution stop. Dans ces conditions, au niveau de tous les brins stoppés à une position donnée, l'élongation reprend. Les complexes arrêtés sont donc toujours actifs.

L'absence de modification des profils de misincorporation en remplaçant le dTTP par le dUTP était prévisible, puisque nous avons observé en l'absence du dATP une seule incorporation d'un T en face d'un T (matrice). La paire T:T asymétrique a été décrite (Donohue. et Trueblood, 1960) mais semble peu stable (Kudritskaya et Danilov V.I., 1976).

Le dITP réduit le taux d'erreur observé avec le dGTP au niveau du premier site de misinsertion de près de 50% au bout de 45 min. d'incubation. La misincorporation en présence de dITP serait donc plus difficile qu'en présence de dGTP. En l'absence de dATP et dGTP, la purine I ne s'apparie à T que dans trois cas, contre 8 appariements T:C et un T:T. Les trois T:I apparaissent d'ailleurs face au premier T-135 (matrice). Nous n'avons pas trouvé de référence sur l'énergie de la paire T:I, mais les taux d'insertions que nous observons sont en faveur du classement T:C>>T:I>>T:T.

L'étude de la fidélité de la reverse transcriptase de HIV-1, effectuée en l'absence d'un des 3 nucléotides, a montré que le taux d'erreur de l'élongation à partir de ppt est plus fort par rapport à la polymérisation à partir de pbs. L'étude, en accord avec celles de Perrino et al., 1989 ; Yu et Goodman, 1992, montre que la reverse transcriptase étend les mésappariements plus facilement qu'elle ne les forme. L'absence de bande de forte terminaison au niveau des sites de misincorporation au-delà du 1^{er} mésappariement suggère que les paires de bases mal appariées n'affectent que peu la poursuite de la polymérisation avec la reverse transcriptase de HIV-1. Dans certains cas, en particulier avec l'amorce ppt, les mésappariements dans le brin néosynthétisé, semblent même favoriser les erreurs suivantes.

L'étude des mutants obtenus en l'absence de dATP lors de la polymérisation catalysée par la reverse transcriptase de HIV-1 indique que les types d'erreur induits par cette reverse transcriptase ne présentent pas de délétions ou d'additions de bases. Globalement l'ordre de substitution aux quatre sites T de la matrice est G»C»T. Il est possible que le mésappariement au niveau du 1^{er} site modifie la spécificité de l'erreur sur les sites suivants, soit en favorisant le déplacement du brin amorce (Fig 4.10), soit en favorisant la dislocation ou soit en modifiant la conformation du site catalytique.

Au niveau du 1^{er} site de misincorporation G est probablement apparié à T en appariement de type « Wobble ». L'appariement T.G de type « Wobble » (Fig 4.9) est stabilisé par des liaisons de type hydrogène avec les 3 molécules d'eau. L'absence de NH2 de I déstabilise cet appariement ce qui expliquerait la diminution de la substitution T :I par rapport à T:G.

La fidélité de la reverse transcriptase de HIV-1, en l'absence de dATP, en présence de dITP au lieu de dGTP est augmenté. La majorité des mutants identifiés en présence de dITP ne présente qu'une mutation de plus l'ordre de substitution, qui est modifié par rapport à la réaction avec dGTP, est C»I»T. Le changement de T par U ne modifie que peu le profil et la spécificité des mutations engendrées par la reverse transcriptase de HIV-1.

### Exemple 6 : Conclusion générale

La processivité et la fidélité de la transcriptase inverse de HIV-1 peuvent être modulées par l'utilisation des bases modifiées.

La présence de bases modifiées dans le mélange réactionnel modifie la processivité au niveau des positions localisées sur les sites d'incorporation des bases modifiées et précédant ces derniers, pouvant impliquer un effet de l'empilement des bases consécutives sur la terminaison de la polymerisation comme nous l'avons montré dans le cas des bases naturelles (résultats non publiés). Dans le cas d'utilisation d'analogues tels que dITP et c7dNTP, nous avons montré des variations de la processivité au niveau de sites non impliqués dans l'incorporation de ces bases modifiées, ces variations peuvent s'expliquer par des modifications de la conformation de la double-hélice ADN impliquant la vingtaine de bases qui est en contact avec l'enzyme. Cette modification de la conformation de la double-hélice est induite par les différences structurales existant entre les bases naturelles et leurs analogues qui conduisent à des changements dans l'interaction entre les bases appariées et les éléments protéiques dans le grand et petit sillon, de la symétrie de l'appariement, des effets stériques, des forces ioniques etc... La double-hélice d'ADN liée à la HIV-1 RT a une conformation caractéristique, une forme A sur les 5 paires de bases près du site catalytique, suivi d'un coude d'environ 40° et une forme B en contact avec le domaine connection (Ding et al., 1997). La modification de la conformation de la double-hélice en contact avec HIV-1 RT induite par la présence de bases modifiées dans le brin néosynthétisé complémenté par le brin matrice va déstabiliser ou stabiliser le complexe réactionnel et respectivement diminuer ou augmenter la processivité.

La présence de bases modifiées peut modifier la fidélité de la transcriptase inverse de HIV-1. *In vitro,* nous avons montré qu'en l'absence de dATP, le remplacement de dGTP par un de ses analogues, le dITP, réduit le taux de mutation, diminue le nombre de mésappariements par plasmide et augmente l'hétérogénicité des plasmides mutés. La réduction du taux de mutation peut s'expliquer par l'absence de NH₂ de I qui déstabilise l'appariement T.I par rapport à T.G, qui est stabilisé par des liaisons de type hydrogène avec 3 molécules d'eau (Fig. 4.9). L'utilisation d'analogues de bases permet ainsi de modifier l'ordre de préférence de mésappariement.

Dans ce qui suit, on se référera aux figures suivantes :
**Figure 1.1** **:** Séquence utilisée dans cette étude. Les amorces sont soulignées. L'amorce pbs est suivie de 16 bases dépourvues de A.

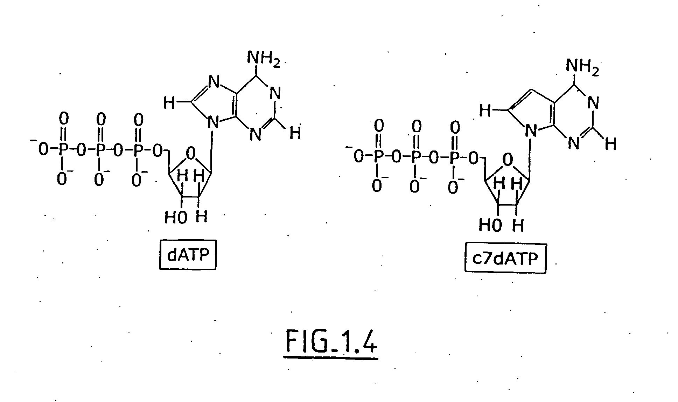

**Figure 1****.****2** **:** Structure chimique du dGTP et de deux de ses analogues le dITP et le c7dGTP.
**Figure 1****.****3** : Structure chimique du dATP et d'un de ses analogues le c7dATP.
**Figure 2****.****1** : Taux de terminaison de la polymérisation catalysée par la reverse transcriptase de HIV-1 en fonction de la séquence à copier. L'abscisse représente la séquence nucléotidique de -env (brin sens) de l'extrémité 3' à 5' qui sert de matrice lors de la polymérisation pour amorce pbs. (□) en présence de dATP, dCTP, dGTP et dTTP,
(□) en présence de dATP, dCTP, dGTP et dUTP à la concentration finale de 500 µM chacun.
**Figure 2.2** **:** Taux de terminaison de la polymérisation catalysée par la reverse transcriptase de HIV-1 en fonction de la séquence à copier. L'abcisse représente la séquence nucléotidique de - env (brin sens) de l'extrémité 3' à 5' qui sert de matrice lors de la polymérisation pour amorce pbs. (□) en présence de dATP, dCTP, dGTP et dTTP,
(□) en présence de dATP, dCTP, dITP et dTTP à la concentration finale de 500 µM chacun.
**Figure 2****.****3** **:** Taux de terminaison de la polymérisation catalysée par la reverse transcriptase de HIV-1 en fonction de la séquence à copier. L'abcisse représente la séquence nucléotidique de - env (brin sens) de l'extrémité 3' à 5' qui sert de matrice lors de la polymérisation pour amorce pbs. (□) en présence de dATP, dCTP, dGTP et dTTP,
(□) en présence de dATP, dCTP, c7dGTP et dTTP à la concentration finale de 500 µM chacun.
**Figure 2****.****4** **:** Taux de terminaison de la polymérisation catalysée par la reverse transcriptase de HIV-1 en fonction de la séquence à copier. L'abcisse représente la séquence nucléotidique de - env (brin sens) de l'extrémité 3' à 5' qui sert de matrice lors de la polymérisation pour amorce pbs. (□) en présence de dATP, dCTP, dGTP et dTTP,
(□) en présence de c7dATP, dCTP, dGTP et dTTP à la concentration finale de 500 µM chacun.
**Figure 2.5** **:** Variation du taux de terminaison avec la reverse transcriptase de HIV-1 avec les différents analogues. L'abscisse représente la séquence nucléotidique du brin sens de l'extrémité 3' à 5'. La variation du taux de terminaison a été calculée en soustrayant le taux de terminaison obtenu entre la réaction effectuée en présence d'un analogue de base (dUTP : bleu ; dITP : rouge ; c7dGTP : vert ; c7dATP noir) aux taux obtenus avec la base normale à chaque site.
**Figure 2.6** **:** Taux de terminaison de la polymérisation catalysée par le fragment de Klenow en fonction de la séquence à copier. L'abscisse représente la séquence nucléotidique - env (brin sens) de l'extrémité 3' à 5' qui sert de matrice lors de la synthèse ayant pour amorce pbs. (□) en présence de dATP, dCTP, dGTP et dTTP, (□) en présence de dATP, dCTP, dGTP et dUTP à la concentration finale de 500 µM chacun.
**Figure 2.7** **:** Taux de terminaison de la polymérisation catalysée par le fragment de Klenow en fonction de la séquence à copier. L'abscisse représente la séquence nucléotidique - env (brin sens) de l'extrémité 3' à 5' qui sert de matrice lors de la synthèse ayant pour amorce pbs. (□) en présence de dATP, dCTP, dGTP et dTTP, (□) en présence de dATP, dCTP, dITP et dTTP à la concentration finale de 500 µM chacun.
**Figure 2.8** **:** Taux de terminaison de la polymérisation catalysée par le fragment de Klenow en fonction de la séquence à copier. L'abscisse représente la séquence nucléotidique - env (brin sens) de l'extrémité 3' à 5' qui sert de matrice lors de la synthèse ayant pour amorce pbs. (□) en présence de dATP, dCTP, dGTP et dTTP, (□) en présence de dATP, dCTP, c7dGTP et dTTP à la concentration finale de 500 µM chacun.
**Figure 2.9** : Taux de terminaison de la polymérisation catalysée par le fragment de Klenow en fonction de la séquence à copier. L'abscisse représente la séquence nucléotidique - env (brin sens) de l'extrémité 3' à 5' qui sert de matrice lors de la synthèse ayant pour amorce pbs. (□) en présence de dATP, dCTP, dGTP et dTTP, (□) en présence de c7dATP, dCTP, dGTP et dTTP à la concentration finale de 500 µM chacun.
**Figure 2.10** **:** Variation du taux de transcription avec le fragment de klenow avec les différents analogues. L'abscisse et représente la séquence nucléotidique du brin sens de l'extrémité 3' à 5'. La variation du taux de terminaison a été calculée en soustrayant le taux de terminaison obtenu entre la réaction effectuée en présence d'un analogue de base (dUTP : bleu ; dITP : rouge ; c7dGTP : vert ; c7dATP : noir) aux taux obtenus avec la base normale à chaque site
**Figure 3****.****1** **:** Variation du taux de terminaison avec la reverse transcriptase de HIV-1, de la reverse transcriptase du virus de la leucémie de Moloney de la souris (MoMLV), de la reverse transcriptase du virus du myeloblastome aviaire (AMV) et du fragment de Klenow en présence de dUTP au lieu de dTTP. L'abscisse représente la séquence nucléotidique du brin sens de l'extrémité 3' à 5'. La variation du taux de terminaison est calculée en soustrayant le taux de terminaison obtenu entre la réaction effectuée en présente du dUTP aux taux obtenus avec la dTTP à chaque site pour chacune des enzymes (HIV-1 RT : bleu ; MoMLV RT : rouge ; AMV : vert ; fragment de Klenow : noir).
**Figure 3****.****2** : Variation du taux de terminaison avec la reverse transcriptase de HIV-1, de la reverse transcriptase du virus de la leucémie de Moloney de la souris (MoMLV), de la reverse transcriptase du virus du myeloblastome aviaire (AMV) et du fragment de Klenow en présence de dITP au lieu de dGTP. L'abscisse représente la séquence nucléotidique du brin sens de l'extrémité 3' à 5'. La variation du taux de terminaison est calculée en soustrayant le taux de terminaison obtenu entré la réaction effectuée en présence du dITP aux taux obtenus avec le dGTP à chaque site pour chacune des enzymes (HIV-1 RT : bleu ; MoMLV RT : rouge ; AMV : vert ; fragment de Klenow : noir).
**Figure 3.3** **:** Variation du taux de terminaison avec la reverse transcriptase de HIV-1, de la reverse transcriptase du virus de la leucémie de Moloney de la souris (MoMLV), de la reverse transcriptase du virus du myeloblastome aviaire (AMV) et du fragment de Klenow en présence de c7dGTP au lieu de dGTP. L'abscisse représente la séquence nucléotidique du brin sens de l'extrémité 3' à 5'. La variation du taux de terminaison est calculée en soustrayant le taux de terminaison obtenu entre la réaction effectuée en présence du c7dGTP aux taux obtenus avec le dGTP à chaque site pour chacune des enzymes (HIV-1 RT : bleu ; MoMLV RT : rouge ; AMV : vert ; fragment de Klenow : noir).
**Figure 3****.****4** **:** Variation du taux de terminaison avec la reverse transcriptase de HIV-1, de la reverse transcriptase du virus de la leucémie de Moloney de la souris (MoMLV), de la reverse transcriptase du virus du myeloblastome aviaire (AMV) et du fragment de Klenow en présence de c7dATP au lieu de dATP. L'abscisse représente la séquence nucléotidique du brin sens de l'extrémité 3' à 5'. La variation du taux de terminaison est calculée en soustrayant le taux de terminaison obtenu entre la réaction effectuée en présence de c7dATP aux taux obtenus avec de dATP à chaque site pour chacune des enzymes (HIV-1 RT : bleu ; MoMLV RT : rouge ; AMV : vert ; fragment de Klenow : noir).
**Tableau 3.1** : Influence de la présence de dIMP par rapport au dGMP dans la séquence du brin néosynthétisé sur la variation du taux de terminaison lors de la réaction catalysée par la reverse transcriptase de HIV-1. La référence 1 est le site de terminaison de la polymérisation considéré.
**Tableau 3.2 :** Influence de la présence de c7dGMP par rapport au dGMP dans la séquence du brin néosynthétisé sur la variation du taux de terminaison lors de la réaction catalysée par la reverse transcriptase de HIV-1. La référence 1 est le site de terminaison de la polymérisation considéré.
**Tableau 3.3 :** Influence de la présence de c7dAMP par rapport au dAMP dans la séquence du brin néosynthétisé sur la variation du taux de terminaison lors de la réaction catalysée par la reverse transcriptase de HIV-1. La référence 1 est le site de terminaison de la polymérisation considéré.
**Figure 4****.****1** **:** Cinétique d'arrêt de polymérisation catalysée par la reverse transcriptase de HIV-1 en présence de dCTP, dGTP et de dTTP à la concentration finale de 500 µM chacun (D) 1 min, (□) 2.5 min, (D) 5 min, (D) 10 min.
**Figure 4****.****2** : Cinétique d'arrêt de polymérisation catalysée par la reverse transcriptase de HIV-1 en présence de dCTP, dGTP et de dUTP à la concentration finale de 500 µM chacun (D) 1 min, (□) 2.5 min, (D) 5 min, (□) 10 min.
**Figure 4****.****3** **:** Cinétique d'arrêt de polymérisation catalysée par la reverse transcriptase de HIV-1 en présence de dCTP, dITP et de dTTP à la concentration finale de 500 µM chacun (□) 1 min, (□) 2.5 min, (□) 5 min, (□) 10 min.
**Figure 4.4** : Taux d'erreur de la reverse transcriptase de HIV-1 au niveau du premier site de misincorporation face à T-135 (matrice + env) en fonction du temps : dCTP, dGTP, dTTP, (◆) dCTP, dGTP, dUTP (■), dCTP, dITP, dTTP (▲), dCTP, dTTP, dUTP (X) à la concentration finale de 500 µM chacun.
**Figure 4.5** **:** Identification des plasmides mutés par digestion enzymatique avec NcoI et SphI lors de la polymérisation avec comme matrice + env et amorcée avec ppt en l'absence de d'ATP.
**Figure 4.6** **:** Mutants obtenus lors de la polymérisation à partir de la matrice + env amorcée par ppt, en l'absence de dATP et en présence de dCTP, dGTP, et dTTP.
   Etude effectuée avec la reverse transcriptase de HIV-1 et du virus de myeloblastome aviaire (AMV).
**Figure 4.7** **:** Mutants obtenus lors de la polymérisation à partir de la matrice + env amorcée par ppt, en l'absence de dATP et en présence de dCTP, dITP, et dTTP. Etude effectuée avec la reverse transcriptase de HIV-1 et du virus de myeloblastome aviaire (AMV).
**Figure 4.8** **:** Mutants obtenus lors de la polymérisation à partir de la matrice + env amorcée par ppt, en l'absence de dATP et en présence de dCTP, dGTP, et dUTP. Etude effectuée avec la reverse transcriptase de HIV-1 et du virus de myeloblastome aviaire (AMV).
**Figure 4.9** **:** Structure du mésappariement Guanine (G) - Thymine (T) de type "WOBBLE". La thymine est projetée dans le grand sillon alors que la guanine est projetée dans le petit sillon de l'ADN. Les 3 molécules d'eau qui stabilisent la paire de base mal appariée grâce à des liaisons de type hydrogène disponibles, sont représentées.
**Figure 4.10** **:** Intermédiaire possible conduisant à la substitution T vers C au niveau du deuxième site de misincorporation. Les bases non complémentaires sont en gras.

### REFERENCES

Abbotts, J., Sen Gupta, D.N., Zon, G. & Wilson, S.H. (1988). Studies on the mechanism of Escherichia coli ADN polymerase I Large Fragment. J Biol. Chem. 263: 15094-15103.
Alkhatib, G., Combadiere, C., Broder, C.C., Feng, Y., Kennedy, P.E., Murphy, P.M. & Berger, E.A. (1996). CC CKR5 : a RANTES, MIP-1 a, MIP-1β receptor as a fusion cofactor for macrophage tropic HIV-1. Science. 272 : 1955-1958.
Benzaria, S., Pélicano, H., Johnson, R., Maury, G., Imbach, J.L., Aubertin, A.M., Obert, G., Gosselin, G. (1996). Synthesis, in vitro antiviral evaluation, and stability studies of bis(S-acyl-2-thioethyl) ester derivatives of 9-[2-(phosphonomethoxy)ethyl]adenine (PMEA) as potential PMEA produgs with improved oral bioavailability. J.Med. Chem. 39 : 4958-4965
Choe, H., Farzan, M., Sun, Y., Sullivan, N., Rollins, B., Ponath, P.D., Wu, L., Mackay, C.R., La Rosa, G., Newman, W., Gerard, N., Gerard, C. & Sodroski, J. (1996) Thé beta-chemokine receptors CCR 3 et CCR5 facilitate infection by primary HIV-1 isolates. Cell. 85 : 1135-1148.
Debyser et De Clercq (1996) J. Immunol. Immunopharmacol. 16 : 48-52.
De Clercq (1997) Int. J. Antimicrob. Agents 9 : 21-36.
De Clercq et Balzarini (1995) Il Farmaco, 50 : 735-747.
Deng, H., Liu, R., Ellmerer, W., Choe, S., Unutmaz, D., Burckhard, M., Di Marzio, P.,Marmon, S., Sutton, R.E., Hill, C.M., Davis, C.B., Peiper, S.C., Schall, T.J., Littaman, D.R. & Landau, N.R. (1996). Identification of a major co-receptor for primary isolates of HIV-1. Nature. 381 :661-666.
Di Marco Veronese, F., Copeland, T.D., DeVico, A.L., Rahman, R., Oroszlan, S., Gallo, R.C. & Sarngadharan, M.G. (1986). Characterization of highly immunogenic p66/p51 as the reverse transcriptase of HTLV-III/LAV. Science. 231 : 1289-1291.
Domingo, E., Martinez-Salas, E., Sobrino, F., de la Torre, J.C., Portela, A., Ortin, J., Lopez-Galindez, C., Perez-Brena, P., Villanueva, N., Najera, R., VandePol, S., Steinhaver, D., Delolo, N. & Holland, J. (1985). The quasispecies (extremely heterogeneous) nature of viral RNA genome populations biological relevance. Gene 40 : 1-8.
Donohue, J. & Trueblood, K.N. (1960). Base pairing in DNA. J. Mol. Biol. 2 363-71.
Fauci, A.S. (1988). The human immunodeficiency virus : infectivity and mechanisms of pathogenesis. Science 239 : 617-622.
Gehrke, C.W. & Kuo, C.T. K. (1985). Analytical methods for major and modified nucleosides in tRNA, mRNA, DNA and physiological fluids : HPLC,GC,MS,NMR,UV and FTIR.Bull. Mol. Biol. Med. 10 : 119-142.
Gojobori, T. & Yokoyama, S. (1985). Rates of évolution of the retroviral oncogene of Moloney murine sarcoma virus and its cellular homologues. Proc. Natl. Acad. Sci. US. 82 : 4198-4201.
Granier et Valantin (1998) Presse médicale 27 : 622-623.
Hill, F., Loakes, D., Brown, D.M. (1998). Polymerase recognition of synthetic oligodeoxyribonucleotides incorporating degenerate pyrimidine and purine bases. Biochemistry. 95 : 4258-4263.
Hu, W.S. & Temin, H.M. (1990b). Retroviral recombination and reverse transcription. Science. 250 : 1227-1233.
Hunter, W.N., Brown, T., Kneale, G., Anand, N.N., Rabinovich, D. & Kennard, O. (1997). The structure of guanosine-thymidine mismatches in B-DNA at 2,5 Å resolution. J. Biol. Chem. 262 :9962-9970.
Japour, A.J., Chatis, P.A., Eigenrauch, H.A &, Crumpacker, C.S. (1991). Detection of human immunodeficiency virus type 1. Clinical isolates with reduced sensitivity to zidovudine and dideoxyinosine by RNA/RNA hybridization. Proc. Natl. Acad. Sci. USA. 88 : 3092-3096.
Ji, J., Hoffmann, J.S. & Loeb, L. (1994). Mutagenicity and pausing of HIV reverse transcriptase during HIV plus-strand DNA synthesis. Nucleic Acids Res. 22 : 47-52.
Ji, J. & Loeb, L.A. (1994). Fidelity of HIV-1 reverse transcriptase copying a hypervariable region of the HIV-1 env gene. Virology 199: 323-330.
Jolly, D.J., Willis, R.C. & Friedmann, T. (1986). Variable stability of a selectable provirus after retroviral vector gene trasnfer into human cells. Mol. Cell. Biol.6 : 1141-1147.
Kim, S.Y., Byrn, R., Groopman, J. & Baltimore, D. (1989). Temporal aspects of DNA and RNA synthesis during human immunodeficiency virus infection : evidence for differential gene expression. J. Virol. 63: 3708-3713.
Kudritskaya, Z.G. & Danilov, V.I. (1976). Quantum mechanical study of base interactions. J. Theor. Biol: 59 303-318.
Lecomte, P.J. & Ninio, J. (1987) Variations with position of replication errors up to exonuclease warm-up. FEBS Lett. 194-198.
Lecomte, P.J. & Ninio, J. (1988). Nucleotide excision by E. coli DNA polymerase I in proofreading and non-proofreading modes. Biochmica Biophysica Acta. 951 : 255-260.
Lefebvre, I., Périgaud, C., Pompon, A., Aubertin, A.M., Girardet, J.L., Kirn, A., Gosselin, G., Imbach, J.L. (1995). Mononucleoside phosphotriester derivatives with S-acyl-2-thioethyl bioreversible phosphate-protecting groups : intracellular delevery of 3'-azido-2',3'-dideoxythymidine 5'-monophosphate. J. Med. Chem. 38 : 3941-3950.
Limbach, P.A., Crain, P.F., Pomerantz, S.C., McCloskey J.A. (1995). Structures of posttranscriptionally modified nucleosides from RNA. Biochimie. 77 : 135-138.
Modrow, S., Hahn, B.H., Shaw, G.M., Gallo, R.C., Wong-Staal, F. & Wolf, H. (1987). Computer-assisted analysis of enveloppe protein sequences of seven human immunodeficiency virus isolates prediction of antigenic epitopes in conserved and variable regions. J. Virol. 61: 570-578.
Morris Jones et al. (1997) Expert. Opin. Invest. Drugs 6(8) : 1049-1061.
Motorin Y., & Grosjean H. (1998). Appendix 1 : chemical structures and classification of posttranscriptionally modified nucleosides in RNA. Modification and Editing of RNA. 543-549.
Papanicolaou, C. & Ripley, L.S. (1989). Polymerase-specific differences in the DNA intermediates of frameshift mutagenesis. In vitro synthesis errors of Escherichia coli DNA polymerase 1 and its large fragment derivative. J. Mol. Biol. 207 : 335-353.
Papanicolaou, C. & Ripley, L.S. (1991) An in vitro approach to identifying specificity determinants of mutagenesis mediated by DNA misalignments. J. Mol. Biol. 221 : 805-821.
Pathak, V.K. & Temin, H.M. (1990a). Broad spectrum of in vivo forward mutations, hypermutations and mutational hotspots in a retroviral shuttle vector after a single replication cycle : deletions and deletions with insertions. Proc. Natl. Acad. Sci. USA. 87: 6024-6028.
Pathak, V.K. & Temin, H.M. (1990b). Broad spectrum of in vivo forward mutations, hypermutations and mutational hot spots in a retroviral shuttle vector after a single replication cycle : substitutions, frameshifts and hypermutations. Proc. Natl. Acad. Sci. USA. 87: 6019-6023.
Patick et al. (1997) Antimicrob. Agents Chemother. 41 : 2159-2164.
Perrino, F.W., Preston, B.D., Sandell, L.L. & Loeb, L.A. (1989). Extension of mismatched 3' termini of DNA is a major determinant of the infidelity of Human Immunodeficiency Virus Type 1 Reverse Transcriptase. Proc. Natl. Acad. Sci. USA. 86: 8343-8347.
Preston, B.D. & Garvey, N. (1992).Pharm. Techn. 16: 34-51.
Saenger, W. (1984). Principles of nucleic acid structure. Springer Verlag NY*.*
Sato, H., Orenstein, L., Dimitrov, D. & Martin, M. (1992). Cell-to-cell spread of HIV-1 occurs within minutes and may not involve the participation of virus particules. Virology 18 : 712-724.
Tong et al. (1997) Biochemistry 36 (19) : 5749-5757.
Wabl, M., Burrows, P.D., Gabian, A.V. & Steinberg, C. (1985). Hypermutation at the immunoglobulin heavy chain locus in a pre-B-cell line. Proc. Natl. Acad. Sci. USA. 82 : 479-482.
Yu, H. & Goodman, M.F. (1992). Comparison of HIV-1 and Avian Myeloblastosis Virus reverse transcriptase fidelity on RNA and DNA templates. J. Biol. Chem. 267: 10888-10896.

## Revendications

1. Utilisation d'analogues de nucléotides pour la réalisation d'un médicament destiné au traitement des affections faisant intervenir la reverse transcriptase et provoquées par les virus rétroïdes **caractérisés en ce que** :
a) lesdits analogues de nucléotides :
- comprennent un motif 2'-désoxyribosyle comprenant en position 3' un groupement OH et en position 5' un groupement phosphate, le dit groupement phosphate étant choisi parmi le groupe constitué par les groupements mono-, di- et tri-phosphates,
et
b) lesdits analogues de nucléotides sont des substrats de la reverse transcriptase, ne terminent pas la réaction de la reverse transcriptase après leur incorporation dans la chaîne naissante, et leur groupement OH en position 3' est capable d'échanger des liaisons phosphodiester avec la chaîne naissante et le nucléotide suivant.

2. Utilisation selon la revendication 1 **caractérisée en ce que** les analogues de nucléotides sont protégés sous la forme de pronucléotides bis-(S-acyl-2-thio-éthyl).

3. Utilisation selon la revendication 1 ou 2, **caractérisée en ce que** lesdits virus rétroïdes infectent l'homme et/ou les animaux et/ou les végétaux.

4. Utilisation selon la revendication 3, **caractérisée en ce que** lesdits virus rétroïdes qui infectent les cellules humaines ou animales appartiennent plus particulièrement au groupe des lentivirus, des oncovirus à ARN, des spumavirus et des hépadnavirus.

5. Utilisation selon la revendication 4, **caractérisée en ce que** le lentivirus est choisi parmi le groupe composé du virus de l'immunodéficience humaine (HIV) et les virus de la leucémie des cellules T humaines (HTLV).

6. Utilisation selon la revendication 5, **caractérisée en ce que** le virus de l'immunodéficience humaine (HIV) est le virus de l'immunodéficience humaine de type 1 (HIV-1).

7. Utilisation selon la revendication 4, **caractérisée en ce que** l'hépadnavirus est le virus de l'hépatite B (HBV).

8. Utilisation d'analogues nucléotides selon l'une quelconque des revendications 1 à 7 **caractérisée en ce que** lesdits analogues de nucléotides sont choisis parmi les nucléotides modifiés présents dans les ARN procaryotes ou eucaryotes.

9. Utilisation d'analogues de nucléotides selon l'une quelconque des revendications 1 à 7, **caractérisée en ce qu'**ils sont choisis parmi le dUTP, le dITP, le C7dGTP et le C7dATP.

10. Utilisation d'analogues de nucléotides selon l'une quelconque des revendications 1 à 9, **caractérisée en ce qu'**ils possèdent les bases modifiées 6H, 8H-3, 4-dihydropyrimido [4,5c][1,2] oxazin-7-one (P) et N⁶-méthoxy-2, 6- diaminopurine (K).

11. Composition pharmaceutique comprenant un véhicule pharmaceutiquement acceptable et des analogues de nucléotides tels que définis dans l'une des revendications précédentes, pour utilisation pour le traitement des affections faisant intervenir la reverse transcriptase et provoquées par les virus rétroïdes selon l'une quelconque des revendications précédentes.

12. Composition pharmaceutique, **caractérisée en ce qu'**elle contient un mélange d'au minimum un analogue de nucléotide selon l'une quelconque des revendications 1 à 10 et d'au minimum un agent antirétroviral choisi parmi le groupe composé des inhibiteurs de la reverse transcriptase nucléotidiques et non nucléotidiques et des antiprotéases virales comme produit de combinaison pour une utilisation simultanée, séparée ou étalée dans le temps en thérapie antivirale.

13. Composition pharmaceutique selon la revendication 12, **caractérisée en ce que** l'inhibiteur de la reverse transcriptase est choisi parmi le 3'-azido- 3'déoxythymidine (AZT), le 2',3'-didéoxyinosine (ddl), le 2',3'-didéoxycytidine (ddC), le (-)2',3' didéoxy-3'-théacytidine (3TC), le 2',3'-didéhydro- 2',3'didéoxythymidine (d4T) et le (-)2'-déoxy-5-fluoro-3'-théacytidine (FTC), le TIBO (Tétrahydroimidazo- (4,5,1-1, jk)(1,4)-benzodiapézine-2(1H)-one), le HEPT (1-[(2-hydroxyéthoxy)-méthyl]-6-phénylthiothymine), le TSAO ([2', 5'-bis-O- (tert-butyldiméthylsilyl)-béta-D ribofuranosyl]-3'-spiro-5"-(4"-amino-1", 2"- oxathiole-2", 2"-dioxide), l'a-APA (alpha-anilinophénylacétamide), la névirapine, le BAHP (bis(étheroaryl)-pipérazine), l'acide phosphonoformique (PFA).

14. Composition pharmaceutique selon la revendication 12, **caractérisée en ce que** l'antiprotéase virale est choisie parmi l'indinavir et le saquinavir.

## Patentansprüche

1. Verwendung von Nukleotidanalogen zur Herstellung eines Arzneimittels zur Behandlung von Zuständen, in welchen die Reverse Transkriptase beteiligt ist und hervorgerufen durch Retroviren, gekennzeichnet in dem:
a) die Nukleotidanalogen eine 2'-Desoxyribosyl Gruppe trägt, welche in der 3'-Stellung mit einer OH-Gruppe und in 5'-Stellung mit einer Phospliatgruppe ausgestattet ist, wobei die Phosphatgruppe beliebig als Mono-, Di- oder Tri-Phosphat ausgewählt werden kann,
und
b) die Nukleotidanalogen sind Substrate für Reverse Transkriptase, welche die Reverse Transkriptase-Reaktion nach ihrer Einarbeitung in die entstehenden Kette nicht anhalten, und deren OH-Gruppe in Stellung 3'die Phosphodiester-Bindung zwischen der entstehenden Kette und dem folgende Nucleotid erlaubt.

2. Verwendung nach Anspruch 1, **dadurch gekennzeichnet, daß** die Nukleotidanalogen in Form geschützter Bis-(S-acyl-2-thio-ethyl) Pronukleotide sind.

3. Verwerdung nach Anspruch 1 oder 2, gekennzeichnet in dem die Viren Menschen und / oder Tieren und / oder Pflanzen infizieren.

4. Verwendung nach Anspruch 3, **dadurch gekennzeichnet, dass** Retroviren, welche menschliche oder tierische Zellen infizieren, insbesondere zu den Gruppen der Lentiviren, RNA Onkoviren, Spumaviren und Hepadnaviren gehören.

5. Verwerdung nach Anspruch 4, **dadurch gekennzeichnet, dass** unter den Lentiviren, die Gruppen der menschlichen Imniundefizienz Viren (HIV) und der menschlichen T-Zell-Leukämie Viren (HTLV) ausgewählt sind.

6. Verwerdung nach Anspruch 5, **dadurch gekennzeichnet, dass** das menschliche Immundefizienz Viren (HIV) das menschliche Immundefizienz Viren Typ 1 (HIV-1) ist.

7. Verwendung nach Anspruch 4, **dadurch gekennzeichnet, dass** der Hepadnavirus der Hepatitis B-Virus (HIV) ist.

8. Die Verwerdung von Nukleotidanalogen nach einem der Ansprüche 1 bis 7, **dadurch gekennzeichnet, dass** die Nukleotidanalogen unter modifizierten Nukleotiden, die in prokaryontischen oder eukaryontischen RNA vorkommen, ausgewählt sind.

9. Die Verwendung von Nukleotidanalogon nach einem der Ansprüche 1 bis 7, **dadurch gekennzeichnet, dass** die Nukleotidanalogen unter dUTP, dITP, c7dGTP und c7dATP ausgewählt sind.

10. Die Verwerdung von Nukleotidnalogen nach einem der Ansprüche 1 bis 9, **dadurch gekennzeichnet, dass** die Nukleotidanalogen die modifizierten Base 6H, 8H-3 ,4-dihydropyrimido [4,5] [1,2]-Oxin 7-on (P) und N6-Methoxy-2,6-Diaminopurin (K), besitzen.

11. Pharmazeutische Zusammensetzung, welche einen pharmazeutisch annehmbaren Träger und Nukleotidanalogen nach irgendeinem der vorhergehenden Anspruche, zwecks Verwerdung zur Behandlung von Krankheiten, welche auf Reverse Transkriptasen ruhen und durch Retroviren hervorgerufen sind.

12. Pharmazeutische Zusammensetzungen, **dadurch gekennzeichnet, daß** sie eine Mischung von mindestens einer Nukleotidanaloge nach einem der Ansprüche 1 bis 10, und mindestens einen retroviralen Wirkstoff, ausgewählt unter den Gruppen der Nukleotid-und Nicht-Nukleotid-Reverse-Transkriptase-Inhibitoren und der viralen Protease-Inhibitoren, für die Anfertigung von. Kombinationspräparate zur gleichzeitige, getrennte oder verzöger antivirale Therapien.

13. Pharmazeutische Zusammensetzung nach Anspruch 12, **dadurch gekennzeichnet, daß** der Auswahl des Reversen Transkriptase Inhibitors 3'-Azido-3'-Desoxythymidin (AZT), 2', 3'-Dideoxyinosin (ddI), 2', 3'-dideoxycitidine (DDC), 2', 3'-Didesoxy-3'-thiacytidin (3TC), 2', 3'-didehydro-2 ', 3'-Didesoxythymidin (d4T) und 2'-Desoxy-5-Fluor-3'- thiacytidin (FTC), TIBO (tetrahydro-(4,5,1-1,jk) (1, 4)-benzodiapezine-2(1H)-on), HETP (1 - [(2 - Hydroxyethoxy) methyl] - 6-phenylthiothymin), TSAO ([2', 5'-Bis-O-(tert-Butyldimethylsilyl)-beta-D-ribofuranosyl]-3'-spiro-5"- (4"-amino-1", 2 "-Oxathiol-2", 2 "-dioxid), alpha-APA (anilinophenylacetamide-alpha), Nevirapin BAHP (bis (etheroaryl) piperazin), und Phosphonoameisensäure (PFA) betrifft.

14. Pharmazeutische Zusammensetzung nach Anspruch 12, **dadurch gekennzeichnet, dass** der virale Protease-Inhibitor Indinavir und Saquinavir ist.

## Claims

1. Use of nucleotide analogues for producing a medicament for the treatment of conditions involving reverse transcriptase, and caused by retroid viruses **characterized in that**:
a) said nucleotide analogs comprise a 2'-deoxyribosyl pattern comprising in 3 'position an OH group and in 5' position a phosphate group, the said phosphate group being selected from the group consisting of mono-, di-and tri -phosphate,
and
b) said nucleotide analogues are substrates for reverse transcriptase, do not stop the reverse transcriptase reaction after their incorporation into the nascent chain, and the OH group in position 3'is able to support the phosphodiester bond formation between the nascent chain and the next nucleotide.

2. Use according to claim 1 **characterized in that** the nucleotide analogs are in the form of protected pronucleotides bis-(S-acyl-2-thio-ethyl),

3. Use according to claim 1 or 2, **characterized in that** said viruses infect humans and / or animals and / or plants.

4. Use according to claim 3, **characterized in that** said retroid viruses that infect human or animal cells belong more particularly to the group of lentiviruses, RNA oncoviruses, spumaviruses and hepadnaviruses.

5. Use according to claim 4, **characterized in that** the lentivirus is selected from the group of the human immunodeficiency virus (HIV) and the virus of human T cell leukemia (HTLV).

6. Use according to claim 5, **characterized in that** the human immunodeficiency virus (HIV) is the human immunodeficiency virus type 1 (HIV 1).

7. Use according to claim 4, **characterized in that** the hepadnavirus is the hepatitis B virus (HIV).

8. The use of nucleoside analogues according to any one of claims 1 to 7 **characterized in that** said nucleotide analogues are selected from modified nucleotides present in prokaryotic or eukaryotic RNA.

9. The use of nucleoside analogue according to any one of claims 1 to 7, **characterized in that** said nucleotide analogues are selected from dUTP, dITP, c7dGTP and c7dATP.

10. The use of nucleoside analogue according to any one of claims 1 to 9 **characterized in that** said analogs have modified nucléotide base 6H, 8H-3 ,4-dihydropyrimido [4,5] [1,2]-oxine 7-one (P) and N6-methoxy-2 ,6-diaminopurine (K).

11. A pharmaceutical composition comprising a pharmaceutically acceptable carrier and nucleotide analogs as defined in the preceding claims for use for the treatment of conditions involving reverse transcriptase and caused by retroid viruses, according to any one of the previous claims.

12. A pharmaceutical composition **characterized in that** it contains a mixture of at least one nucleotide analogue according to any one of claims 1 to 10 and at least one retroviral agent selected from the group consisting of nucleotide and non-nucleotide reverse transcriptase inhibitors and viral protease inhibitors as combination product for simultaneous, separate or delayed in antiviral therapy.

13. A pharmaceutical composition according to claim 12, **characterized in that** the reverse transcriptase inhibitor is selected from 3'-azido-3'-déoxythimidine (AZT), 2 ', 3'-dideoxyinosine (ddI), 2' , 3'-didéoxycitidine (ddC), 2 ', 3'-dideoxy-3'-thiacytidine (3TC), 2', 3'-didehydro-2', 3'-dideoxythymidine (d4T) and 2'- deoxy-5-fluoro-3'-thiacytidine (FTC), TIBO (tetrahydroimidazo-(4,5,1-1,jk) (1, 4)-benzodiapezine-2 (1H)-one), the HETP (1-[(2 - hydroxyethoxy) methyl]-6-phénylthiothymine), the TSAO ([2 ', 5'-bis-O-(tert-butyldimethylsilyl)-beta-D-ribofuranosyl]-3'-spiro-5"-(4"-amino-1", 2"-oxathiole-2", 2"-dioxide), alpha-APA (anilinophénylacetamide-alpha), and nevirapine BAHP (bis (étheroaryl) piperazine), the 'phosphonoformic acid (PFA).

14. A pharmaceutical composition according to claim 12, **characterized in that** the viral protease inhibitor is selected from indinavir and saquinavir.
